# EUROPEAN PATENT APPLICATION

(11) **EP 2 896 620 A1**
(43) Date of publication of application: **22.07.2015**
(21) Application number: 13837393.1
(22) Date of filing: 12.09.2013
(51) Int. Cl.: C07D 417/12, C07D 471/02, A61K 31/4745, A61K 31/519, A61K 31/501, A61P 35/00

(54) **ALKYNYL HETEROAROMATIC RING COMPOUND AND APPLICATION THEREOF**

(30) Priority: 17.09.2012 CN 201210344248
(71) Applicant: Nanjing Sanhome Pharmaceutical Co., Ltd., Nanjing Jiangsu 210038 (CN)
(72) Inventor: WANG, Yong, Nanjing Jiangsu 210038 (CN); ZHAO, Liwen, Nanjing Jiangsu 210038 (CN); ZHANG, Wenping, Nanjing Jiangsu 210038 (CN); CHEN, Hongyan, Nanjing Jiangsu 210038 (CN); BI, Sheng, Nanjing Jiangsu 210038 (CN); GAO, Yiping, Nanjing Jiangsu 210038 (CN); CHEN, Hongbin, Nanjing Jiangsu 210038 (CN); LIU, Yang, Nanjing Jiangsu 210038 (CN); XU, Xin, Nanjing Jiangsu 210038 (CN); ZHANG, Cang, Nanjing Jiangsu 210038 (CN)
(74) Representative: Laufhütte, Dieter
(86) International application number: PCT/CN2013/083402
(87) International publication number: WO 2014/040549

(57) **Abstract**

The present invention belongs to the field of pharmaceutical chemistry, and specifically relates to compounds having an alkynyl heteroaromatic ring structure and pharmaceutically acceptable salts, stereoisomers, N-oxides, solvates, or prodrugs thereof, and pharmaceutical compositions comprising these compounds, as well as uses of these compounds and compositions in the manufacture of a medicament. The compounds of the present invention and the pharmaceutically acceptable salts, stereoisomers, N-oxides, solvates or prodrugs thereof and the pharmaceutical compositions comprising the compounds have better anti-tumor activity.

## Description

### Technical Field

The present invention belongs to the field of pharmaceutical chemistry, and specifically relates to compounds having an alkynyl heteroaromatic ring structure and pharmaceutically acceptable salts, stereoisomers, N-oxides, solvates, crystals, or prodrugs thereof, and pharmaceutical compositions comprising these compounds, as well as uses of these compounds or compositions in the manufacture of a medicament.

### Background

Protein tyrosine kinases (PTKs) are a class of proteases capable of catalyzing the phosphorylation of the phenolic hydroxyl groups of tyrosine residues in various proteins and thus activating functions of functional proteins. Tyrosine kinases (PTKs) account for about one half of the protein kinases in human body, and play very important roles in the intracellular signal transduction pathways, and regulate a series of physiological and biochemical processes such as cell growth, differentiation and death. Studies show that activation sites of more than half of proto-oncogenes and oncogenes are associated with PTKs. Abnormal expression of PTKs can cause disorder of cell proliferation regulation, and further result in tumorigenesis. In addition, abnormal expression of tyrosine kinases is also closely associated with invasion and metastasis of tumors, angiogenesis in tumors and chemotherapy resistance of tumors.

Tyrosine kinase inhibitors can be used as a competitive inhibitor of adenosine triphosphate (ATP) binding to tyrosine kinase, and can competitively bind to tyrosine kinases, block the activity of tyrosine kinases and inhibit cell proliferation. Several protein tyrosine kinase inhibitors have been successfully developed.

Imatinib is the first molecular targeted PTKs inhibitor, and is used for treating various types of cancers and is also used for treating atherosclerosis, thrombosis, restenosis or fibrosis. Clinically, Imatinib is used in its mesylate salt form, with the trade name Gleevec. Imatinib competitively inhibits the binding sites of adenosine triphosphate (ATP) to thymidine kinase (TK) receptors such as KIT, and prevents phosphorylation of TK, thereby inhibiting the signal transduction. Imatinib can inhibit the KIT mutation associated with kinase and the wild type KIT. There are three main targets for Imatinib: ABL protein, KIT protein and platelet-derived growth factor (PDGF) receptor. Therefore, Imatinib can inhibit Bcr-Abl tyrosine kinases at the cellular level *in vivo* and *in vitro,* and selectively inhibit proliferation and induce apoptosis the cells of Bcr-Abl positive cell lines as well as fresh leukemic cells from patients of Philadelphia chromosome-positive (Ph+) chronic myeloid leukemia (CML) and acute lymphoblastic leukemia. In addition, Imatinib can also inhibit receptor tyrosine kinases for platelet-derived growth factor (PDGF) and stem cell factor (SCF), c-Kit, thereby inhibiting PDGF and stem cell factor-mediated cellular behaviors. Clinically, Imatinib is mainly used in treatment of patients with CML in accelerated phase, blast crisis or chronic phase after failure of α-interferon therapy, and patients with unresectable or metastatic malignant gastrointestinal stromal tumor (GIST), and is also used to treat CD117-positive GIST.

Development and clinical use of Imatinib opens a new era of molecular targeted tumor therapy. However, long-term use of Imatinib may cause drug resistance, resulting in tumor recurrence. With wide clinical use of Imatinib, problem of drug resistance has become increasingly prominent. Some CML patients have natural tolerance (drug resistance) to Imatinib, and the other patients respond to Imatinib during the initial phase of drug therapy, but acquired drug-resistance gradually occurs during the course of treatment. The acquired tolerance (drug-resistance) is generated mainly because that Imatinib is unable to bind to Bcr-Abl due to Bcr-Abl point mutations. Furthermore, it has been found that hundreds of Bcr-Abl point mutations are associated with Imatinib resistance, of which 15% to 20% of Imatinib-resistant patients have T315I mutation. Emergence of Imatinib resistance arouses the research upsurge of a new generation of tyrosine kinase inhibitors.

AP24534 developed by Ariad Pharmaceuticals, Inc. (as shown in Formula A) well addresses this problem. Research shows that AP24534 is effective for CML patients having T315I mutation and resistant to second-generation TKIs, and is a multi-targeted kinase inhibitor against Bcr-Abl and SRC. AP24534 may act on the wild type cells and T315I-mutated cells, and inhibit Bcr-Abl and all mutants thereof including the T315I variants resistant to various therapeutic drugs, and is a broad spectrum inhibitor of Bcr-Abl.

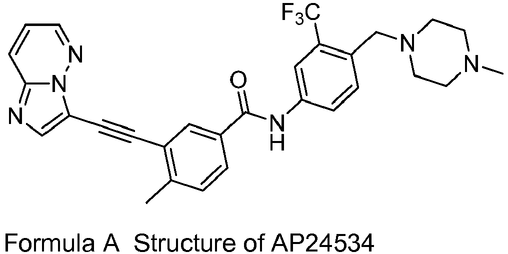

### Summary

The main objective of the present invention is to develop a class of novel protein kinase inhibitors having an alkynyl heteroaromatic structure, which are the protein kinase inhibitors capable of having multi-targeted effects on Bcr-Abl and SRC and having good activity against drug resistant enzymes resulted from T315I mutations.
To achieve the above objectives, in a first aspect, the present invention provides a compound of general formula I or a pharmaceutically acceptable salt, stereoisomer, N-oxide, solvate, crystal, or prodrug thereof, 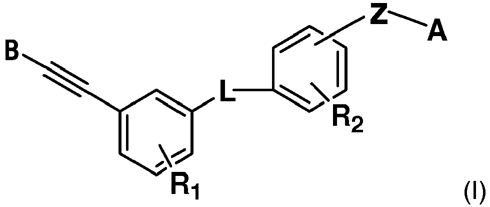 Wherein
L is selected from -C(O)NH-, -NHC(O)NH- and -NHC(O)-;
Z is selected from (CH₂)ₙ and O, wherein n is selected from 0, 1, 2, 3 and 4;
A is selected from substituted and unsubstituted 5-, 6- and 7-membered nitrogen-containing heterocyclic groups;
R₁ is selected from H, alkyl, alkoxy, halo-substituted alkyl, halo-substituted alkoxy, -OH, - NH₂, halogen and -CN;
R₂ is selected from H, alkyl, alkoxy, halo-substituted alkyl, halo-substituted alkoxy, -OH, - NH₂, halogen and -CN; and
B is selected from groups represented by the formula 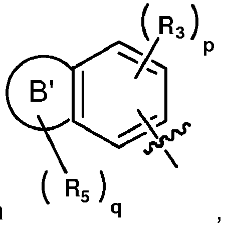
wherein B' is a 5- to 7-membered saturated or unsaturated heteroaromatic ring containing 1 to 3 nitrogen atoms;
R₃ is selected from H, alkyl, alkoxy, halo-substituted alkyl, halo-substituted alkoxy, -OH, - NH₂, halogen and -CN;
R₅ is selected from H, -NR₆R₇, -NHCOR₈, -SO₂R₈, alkyl, alkoxy, halo-substituted alkyl, halo-substituted alkoxy, -OH, halogen, oxo, -CN, hydrazino and alkyl substituted hydrazino, wherein R₆ and R₇ are independently selected from H, alkyl, 4-methylsulphonylanilino and 4-aminosulphonylanilino, and R₈ is selected from H and alkyl;
p is selected from 1, 2 and 3; and
q is selected from 1 and 2.

In some preferred embodiments, the present invention provides a compound of general formula I, or a pharmaceutically acceptable salt, stereoisomer, N-oxide, solvate, crystal, or prodrug thereof, wherein A is selected from piperazinyl, pyridinyl, imidazolyl, pyrrolidinyl, piperidinyl, morpholinyl, thiomorpholinyl, 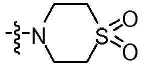 or substituted piperazinyl, pyridinyl, imidazolyl, pyrrolidinyl, piperidinyl, morpholinyl, thiomorpholinyl, wherein the substituent(s) is(are) selected from alkyl, hydroxy, hydroxyalkyl, alkoxy, amino, mono-alkylamino, dialkylamino, amido, alkylamido, arylamido, heteroarylamido, halogen, halo-substituted alkyl, halo-substituted alkoxy and -CN, and preferably, the substituent(s) is(are) selected from C₁₋₆ alkyl, hydroxy, hydroxy C₁₋₆ alkyl, C₁₋₆ alkoxy, amino, mono-C₁₋₆ alkyl, di-C₁₋₆ alkylamino, amido, C₁₋₆ alkylamido, arylamido, heteroarylamido, halogen, halo-substituted C₁₋₆ alkyl, halo-substituted C₁₋₆ alkoxy and -CN.

In some preferred embodiments, the present invention provides a compound of general formula I, or a pharmaceutically acceptable salt, stereoisomer, N-oxide, solvate, crystal, or prodrug thereof, wherein when Z is O, A is pyridinyl or pyridinyl substituted by N-alkyl substituted carboxamido, preferably, A is pyridinyl, N-methylpicolinamido, N-ethylpicolinamido, N-propylpicolinamido; and further preferably, A is pyridin-4-yl, N-methylpicolinamido-4-yl, N-ethylpicolinamido-4-yl, N-picolinamido-4-yl; when Z is selected from (CH₂)ₙ ( wherein n is selected from 0, 1, 2 and 3), A is selected from morpholinyl, thiomorpholinyl, 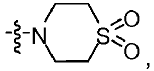 1 H-imidazolyl, 4-methyl-1H-imidazolyl, piperidinyl, piperazinyl, pyrrolidinyl, and piperazinyl, pyrrolidinyl and piperidinyl substituted by one or more amino groups, C₁₋₃ alkyl groups, bi-C₁₋₃ alkylamino groups, mono-C₁₋₃ alkylamino groups or hydroxyethyl groups, and preferably A is selected from morpholinyl, thiomorpholinyl, 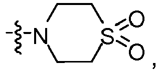 1H-imidazolyl, 4-methyl-1 H-imidazolyl, piperazinyl, 4-methylpiperazinyl, 4-dimethylaminopiperazinyl, 4-methylaminopiperazinyl, 4-hydroxyethylpiperazinyl, 4-dimethylaminoethylpiperazinyl, piperidinyl, 4-dimethylaminopiperidinyl, pyrrolidinyl, 3-aminopyrrolidinyl, (R)-3-aminopyrrolidinyl, (S)-3-aminopyrrolidinyl, 3-dimethylaminopyrrolidinyl, (R)-3-dimethylaminopyrrolidinyl, (S)-3-dimethylaminopyrrolidinyl, 3-methylaminomethylpyrrolidinyl and 3-amino-3-methylpyrrolidinyl.

In other preferred embodiments, the present invention provides a compound of general formula I, or a pharmaceutically acceptable salt, stereoisomer, N-oxide, solvate, crystal, or prodrug thereof, wherein B' is a 5-, 6- or 7-membered heteroaromatic ring containing 2 or 3 nitrogen atoms, for example, pyrazole, imidazole, triazole, pyridazine, pyrimidine, pyrazine and triazine, and further preferably, B' is a 5-, 6- or 7-membered heteroaromatic ring containing 2 or 3 nitrogen atoms, for example, pyrazole, imidazole, triazole, pyridazine, pyrimidine and pyrazine.

In further preferred embodiments, the present invention provides a compound of general formula I, or a pharmaceutically acceptable salt, stereoisomer, N-oxide, solvate, crystal, or prodrug thereof, wherein:
L is selected from -C(O)NH-, -NHC(O)NH- and -NHC(O)-;
Z is selected from (CH₂)ₙ and O, wherein n is selected from 0, 1, 2 and 3;
when Z is O, A is pyridinyl or pyridinyl substituted by N-alkyl substituted carboxamido, preferably, A is pyridinyl, N-methylpicolinamido, N-ethylpicolinamido, N-propylpicolinamido, and further preferably, A is pyridin-4-yl, N-methylpicolinamido-4-yl, N-ethylpicolinamido-4-yl or N-propylpicolinamido-4-yl;
when Z is selected from (CH₂)ₙ (wherein n is selected from 0, 1, 2 and 3), A is selected from morpholinyl, thiomorpholinyl, 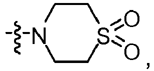 1 H-imidazolyl, 4-methyl-1 H-imidazolyl, piperidinyl, piperazinyl, pyrrolidinyl, and piperazinyl, pyrrolidinyl and piperidinyl substituted by one or more amino groups, C₁₋₃ alkyl groups, bi-C₁₋₃ alkylamino groups, mono-C₁₋₃ alkylamino groups or hydroxyethyl groups, and preferably A is selected from morpholinyl,
thiomorpholinyl, 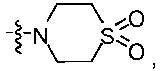 1 H-imidazolyl, 4-methyl-1H-imidazolyl, piperazinyl, 4-methylpiperazinyl, 4-dimethylaminopiperazinyl, 4-methylaminopiperazinyl, 4-hydroxyethylpiperazinyl, 4-dimethylaminoethylpiperazinyl, piperidinyl, 4-dimethylaminopiperidinyl, pyrrolidinyl, 3-aminopyrrolidinyl, (R)-3-aminopyrrolidinyl, (S)-3-aminopyrrolidinyl, 3-dimethylaminopyrrolidinyl, (R)-3-dimethylaminopyrrolidinyl, (S)-3-dimethylaminopyrrolidinyl, 3-methylaminomethylpyrrolidinyl and 3-amino-3-methylpyrrolidinyl;
R₁ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN, and preferably R₁ is selected from H, methyl, ethyl, propyl, isopropyl, C₁₋₃ alkoxy, halo-substituted C₁₋₃ alkyl, halo-substituted C₁₋₃ alkoxy, -OH, -NH₂, fluoro, chloro, bromo, and -CN;
R₂ is selected from H, C₁₋₃ alkyl, C₁₋₆ alkoxy, halo-substituted C₁₋₆ alkyl, halo-substituted C₁₋₆ alkoxy, -OH, -NH₂, halogen and -CN, preferably R₂ is selected from H, C₁₋₃ alkyl, C₁₋₃ alkoxy, halo-substituted C₁₋₃ alkyl, halo-substituted C₁₋₃ alkoxy, -OH, -NH₂, fluoro, chloro and -CN, and more preferably R₂ is selected from H, C₁₋₃ alkyl, C₁₋₃ alkoxy, chloromethyl, fluoromethyl, dichloromethyl, difluoromethyl, trichloromethyl, trifluoromethyl, chloroethyl, fluoroethyl, dichloroethyl, difluoroethyl, trichloroethyl, trifluoroethyl, dichloromethoxy, difluoromethoxy, trichloromethoxy, trifluoromethoxy, dichloroethoxy, difluoroethoxy, trichloroethoxy, trifluoroethoxy, -OH, -NH₂, fluoro, chloro and -CN; and
B is selected from groups represented by the formula 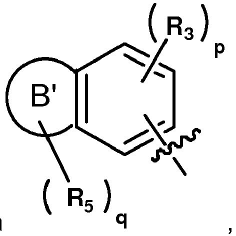 wherein B' is a 5- to 7-membered saturated or unsaturated heteroaromatic ring containing 2 or 3 nitrogen atoms;
R₃ is selected from H, C₁₋₆ alkyl, C₁₋₆ alkoxy, halo-substituted C₁₋₆ alkyl, halo-substituted C₁₋₆ alkoxy, -OH, -NH₂, fluoro, chloro and -CN;
R₅ is selected from H, -NR₆R₇, -NHCOR₈, -SO₂R₈, C₁₋₆ alkyl, C₁₋₆ alkoxy, halo-substituted C₁₋₆ alkyl, halo-substituted C₁₋₆ alkoxy, -OH, halogen, oxo, -CN, hydrazino and C₁₋₆ alkyl substituted hydrazino, wherein R₆ and R₇ are independently selected from H, C₁₋₆ alkyl, 4-methylsulphonylanilino and 4-aminosulphonylanilino, and R₈ is selected from H and C₁₋₆ alkyl, and preferably R₅ is selected from H, C₁₋₃ alkyl, C₁₋₃ alkoxy, halo-substituted C₁₋₃ alkyl, halo-substituted C₁₋₃ alkoxy, C₁₋₃ alkylamido, C₁₋₃ alkylamino, -OH, halogen, oxo, -CN, hydrazino and C₁₋₃ alkyl substituted hydrazino;
p is 1 or 2; and
q is 1.
In a second aspect, the present invention provides a compound of general formula I or a pharmaceutically acceptable salt, stereoisomer, N-oxide, solvate, crystal, or prodrug thereof, 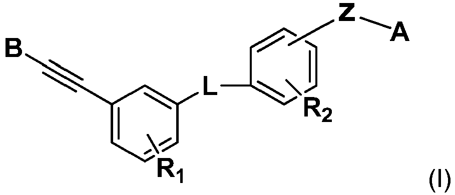 wherein
L is selected from -C(O)NH-, -NHC(O)NH- and -NHC(O)-;
Z is selected from (CH₂)ₙ and O, wherein n is selected from 0, 1, 2, 3 and 4;
A is selected from substituted and unsubstituted 5-, 6- and 7-membered nitrogen-containing heterocyclic groups;
R₁ is selected from H, alkyl, alkoxy, halo-substituted alkyl, halo-substituted alkoxy, -OH, - NH₂, halogen and -CN;
R₂ is selected from H, alkyl, alkoxy, halo-substituted alkyl, halo-substituted alkoxy, -OH, - NH₂, halogen and -CN; and
B is selected from the following structures: 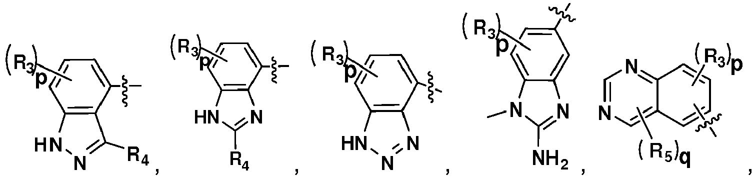 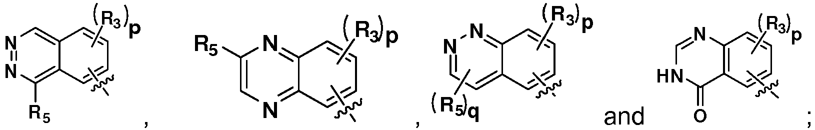
wherein R₃ is selected from H, alkyl, alkoxy, halo-substituted alkyl, halo-substituted alkoxy, -OH, -NH₂, halogen and -CN;

   R₄ is -NH₂;
R₅ is selected from H, -NR₆R₇, -NHCOR₈, -SO₂R₈, alkyl, alkoxy, halo-substituted alkyl, halo-substituted alkoxy, -OH, halogen, -CN, hydrazino and alkyl substituted hydrazino, wherein R₆ and R₇ are independently selected from H, alkyl, 4-methylsulphonylanilino and 4-aminosulphonylanilino, and R₈ is selected from H and alkyl;
p is selected from 1, 2 and 3; and
q is selected from 1 and 2.

In the meantime, the present invention also relates to a compound of general formula I and a pharmaceutically acceptable salt thereof, wherein the pharmaceutically acceptable salt is a salt formed with the following acids: phosphoric acid, sulfuric acid, hydrochloric acid, hydrobromic acid, nitric acid, citric acid, maleic acid, malonic acid, mandelic acid, succinic acid, fumaric acid, acetic acid, lactic acid, sulfonic acid, oxalic acid, tartaric acid, p-toluenesulfonic acid, methanesulfonic acid, camphorsulfonic acid, gluconic acid, malic acid, palmitic acid, trifluoroacetic acid or an amino acid.

In a third aspect, the present invention relates to a pharmaceutical composition comprising a therapeutically effective amount of the compound of general formula I or a pharmaceutically acceptable salt, stereoisomer, N-oxide, solvate or prodrug thereof, and a pharmaceutically acceptable carrier.

In a fourth aspect, the present invention relates to a use of the compound of general formula I or a pharmaceutically acceptable salt, stereoisomer, N-oxide, solvate or prodrug thereof in the manufacture of a medicament for treating or preventing tumors. The tumors include leukemia, non-small cell lung cancer, small cell lung cancer, lung adenocarcinoma, lung squamous carcinoma, histiocytic lymphoma, gastrointestinal stromal tumor, pancreatic cancer, prostate cancer, breast cancer, ovarian cancer, nasopharyngeal cancer, skin cancer, epithelial cell cancer and osteosarcoma.

The term "alkyl" in the present invention refers to a straight-chain or branched-chain saturated hydrocarbon radical, and preferably is C₁₋₆ alkyl, further preferably is C₁₋₅ alkyl, and more preferably is C₁₋₃ alkyl. A suitable C₁₋₃ alkyl is methyl, ethyl, propyl or cyclopropyl. The term "alkoxy" in the present invention refers to an alkyl-O- group, and preferably is C₁₋₆ alkoxy, further preferably is C₁₋₅ alkoxy, and more preferably is C₁₋₃ alkoxy. A suitable C₁₋₃ alkoxy is methoxy, ethoxy, propoxy or isopropoxy.

The term "halogen" in the present invention refers to fluoro, chloro, bromo or iodo.

The term "halo-substituted alkyl" in the present invention refers to an alkyl group substituted by at least one halogen, and preferably is halo-substituted C₁₋₆ alkyl, further preferably is halo-substituted C₁₋₅ alkyl, and more preferably is halo-substituted C₁₋₃ alkyl. A suitable halo-substituted C₁₋₃ alkyl is chloromethyl, fluoromethyl, dichloromethyl, difluoromethyl, trichloromethyl, trifluoromethyl, chloroethyl, fluoroethyl, dichloroethyl, difluoroethyl, trichloroethyl or trifluoroethyl.

The term "halo-substituted alkoxy" in the present invention refers to an alkoxy group substituted by at least one halogen, and preferably is halo-substituted C₁₋₆ alkoxy, further preferably is halo-substituted C₁₋₅ alkoxy, and more preferably is halo-substituted C₁₋₃ alkoxy. A suitable halo-substituted C₁₋₃ alkoxy is dichloromethoxy, difluoromethoxy, tri-chloromethoxy, trifluoromethoxy, dichloroethoxy, difluoroethoxy, trichloroethoxy or trifluoroethoxy.

The term "5-, 6- and 7-membered nitrogen-containing heterocyclic groups" in the present invention refers to a substituted or unsubstituted heterocyclic group that is saturated, partially saturated and fully unsaturated and has at least one ring and the total number of five, six or seven ring atoms wherein at least one ring atom is nitrogen atom. The nitrogen-containing heterocyclic group in the present invention may additionally contain one or more other heteroatoms such as O and S. Preferably, the "5-, 6- and 7-membered nitrogen-containing heterocyclic groups" is morpholinyl, thiomorpholinyl, 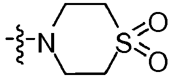 1H-imidazolyl, 4-methyl-1 H-imidazolyl, piperidinyl, piperazinyl, pyrrolidinyl or pyridinyl, wherein the piperazinyl, pyrrolidinyl, piperidinyl may be substituted by one or more amino groups, C₁₋₃ alkyl groups, bi-C₁₋₃ alkylamino groups, mono-C₁₋₃ alkylamino groups and hydroxyethyl groups, and the pyridinyl may be substituted by N-alkyl substituted carboxamido. Preferably, the N-alkyl substituted carboxamido substituted pyridinyl is N-methylpicolinamido, N-ethylpicolinamido or N-propylpicolinamido; and further preferably, the pyridinyl is pyridin-4-yl, and the N-alkyl substituted carboxamido substituted pyridinyl is N-methylpicolinamido-4-yl, N-ethylpicolinamido-4-yl or N-propylpicolinamido-4-yl. Suitable groups include morpholinyl, thiomorpholinyl, 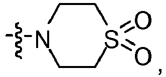 1 H-imidazolyl, 4-methyl-1H-imidazolyl, N-methylpicolinamido, piperazinyl, 4-methylpiperazinyl, 4-dimethylaminopiperazinyl, 4-methylaminopiperazinyl, 4-hydroxyethylpiperazinyl, 4-dimethylaminoethylpiperazinyl, piperidinyl, 4-dimethylaminopiperidinyl, pyrrolidinyl, 3-aminopyrrolidinyl, (R)-3-aminopyrrolidinyl, (S)-3-aminopyrrolidinyl, 3-dimethylaminopyrrolidinyl, (R)-3-dimethylaminopyrrolidinyl, (S)-3-dimethylaminopyrrolidinyl, 3-methylaminomethylpyrrolidinyl and 3-amino-3-methylpyrrolidinyl.

As used herein, the term "5- to 7-membered saturated or unsaturated heteroaromatic ring containing 1 to 3 nitrogen atoms" refers to a saturated, partially saturated or fully unsaturated 5- to 7-membered heteroaromatic ring system containing 1 to 3 nitrogen atoms, in particular 5- to 7-membered saturated and partially unsaturated heteroaromatic ring containing 2 or 3 nitrogen atoms, for example, pyrazole, imidazole, triazole, pyridazine, pyrimidine, pyrazine and triazine. As used herein, the "5- to 7- membered saturated or unsaturated heteroaromatic ring containing 1 to 3 nitrogen atoms" is fused to a benzene ring.

The term "solvate" in the present invention in the conventional sense refers to a complex formed by coordination of a solute (for example, an active compound or a salt of the active compound) with a solvent (for example, water). The solvent is a solvent known or readily determined by a person skilled in the art. When the solvent is water, the solvate is usually referred to as a hydrate, for example, monohydrate, dihydrate or trihydrate.

The term "stereoisomer" in the present invention refers to the R or S configuration of a compound. Accordingly, the individual stereo-chemical isomers and the mixtures of the enantiomers of the present invention are within the scope of the present invention.

The term "chemically protected form" in the present invention refers to a compound in which one or more reactive functional groups are protected from undesired chemical reactions, that is, are in the form of a protected or protecting group. By protecting a reactive functional group, reactions involving other unprotected reactive functional groups can be performed, without affecting the protected group; the protecting group is usually removed in a subsequent step, without substantially affecting the remainder of the molecule.

The term "prodrug" in the present invention refers to a compound which is converted into the compound of general formula I by reacting with enzymes, gastric acid and the like in the physiological condition in the living body, that is, a compound which is converted into the compound of general formula I via enzymatic oxidation, reduction, hydrolysis, and a compound which is converted to the compound of general formula I via hydrolysis in gastric acid and the like.

The term "pharmaceutical composition" in the present invention refers to a mixture comprising any one of the compounds described herein (or corresponding isomers, prodrugs, solvates, pharmaceutically acceptable salts or chemically protected form thereof) and one or more pharmaceutically acceptable carriers and/or excipients. The purpose of using a pharmaceutical composition is to facilitate administration of a compound to a living body. Typically, the composition is used for the treatment and/or prevention of the diseases or undesirable conditions mediated by one or more kinases, wherein the kinases are inhibited by the compounds of the present invention.

The term "pharmaceutically acceptable carrier" in the present invention refers to a carrier which does not cause significant irritation to an organism and does not interfere with the biological activity and properties of the administered compound, including any and all solvents, diluents or other excipients, dispersants, surfactants, isotonic agents, thickening agents or emulsifying agents, preservatives, solid binders, lubricants and the like, except for any conventional carrier medium which is incompatible with the compounds of the present invention. Examples of the pharmaceutically acceptable carrier include, but are not limited to, saccharides such as lactose, glucose and sucrose; starches such as corn starch and potato starch; cellulose and its derivatives such as sodium carboxymethyl cellulose as well as cellulose and cellulose acetate; malt, gelatin and the like.

The term "excipient" in the present invention refers to an inert substance which is added to the pharmaceutical composition of the present invention to further administer the compound. The excipients may include calcium carbonate, calcium phosphate, various sugars and various types of starch, cellulose derivatives, gelatin, vegetable oils and polyethylene glycols.

The expression "use in the manufacture of a medicament for treating or preventing tumors" in the present invention refers to inhibiting the growth, development and/or metastasis of cancers, mainly administering a therapeutically effective amount of the compound of the present invention to a human or animal in need thereof to inhibit, slow or reverse the growth, development or spread of tumors in the subject, wherein the tumors include solid tumors such as breast cancer, ovarian cancer, osteosarcoma, colon cancer, pancreatic cancer, CNS cancer, head and neck cancer, and various types of leukemia and other cancers such as non-small cell lung cancer, small cell lung cancer, lung adenocarcinoma, lung squamous carcinoma, histiocytic lymphoma, gastrointestinal stromal tumors, prostate cancer, nasopharyngeal cancer, skin cancer, and epithelial cell cancer.

The term "pharmaceutically acceptable derivatives" in the present invention refers to any pharmaceutically acceptable salts or esters of the compounds or salts of the esters, or any other adducts or derivatives of the compounds, wherein the pharmaceutically acceptable derivatives also include prodrugs. The compounds of the invention can effectively inhibit the growth of various tumor cells, and have inhibitory effect on proteases such as Bcr-Abl, c-Kit and PDGF. Accordingly, the compounds of the present invention can be used to treat hyperproliferative diseases such as tumors. The compounds of the invention may be used in combination with one or more other known drugs for treating or ameliorating similar disorders.

Unless otherwise stated, the drugs in combination with the compounds of the invention include, but are not limited to, protein tyrosine inhibitors, EGFR inhibitors, VEGFR inhibitors, Bcr-Abl inhibitors, c-kit inhibitors, c-Met inhibitors, Raf inhibitors, MEK inhibitors, Histone deacetylase inhibitors, VEGF antibodies, EGF antibodies, HIV protein kinase inhibitors, and HMG-CoA reductase inhibitors.

The drugs or active ingredients for combination use include, but are not limited to, interferon, alendronate, aclarubicin, platinum drugs, capecitabine, daunorubicin, 5-fluorocytidine and imatinib mesylate.

In some preferred embodiments of the present invention, the groups in general formula I have each independently preferably the following definition:
L is selected from -C(O)NH-, -NHC(O)NH- and -NHC(O)-;
Z is selected from (CH₂)ₙ and O, wherein n is selected from 0, 1, 2 and 3;
when Z is O, A is pyridinyl or pyridinyl substituted by N-alkyl substituted carboxamido, preferably, A is pyridinyl, N-methylpicolinamido, N-ethylpicolinamido, N-propylpicolinamido; and further preferably, A is pyridin-4-yl, N-methylpicolinamido-4-yl, N-ethylpicolinamido-4-yl, or N-propylpicolinamido-4-yl;
when Z is selected from (CH₂)ₙ (wherein n is selected from 0, 1, 2 and 3), A is selected
from morpholinyl, thiomorpholinyl, 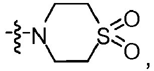 1 H-imidazolyl, 4-methyl-1 H-imidazolyl, piperidinyl, piperazinyl, pyrrolidinyl, and piperazinyl, pyrrolidinyl and piperidinyl substituted by one or more amino groups, C₁₋₆ alkyl groups, bi-C₁₋₆ alkylamino groups, mono-C₁₋₆ alkylamino groups or hydroxyethyl groups, and preferably A is selected from piperazinyl, pyrrolidinyl and piperidinyl by one or more amino groups, C₁₋₃ alkyl groups, bi-C₁₋₃ alkylamino groups, mono-C₁₋₃ alkylamino groups or hydroxyethyl groups, and more preferably A
is selected from morpholinyl, thiomorpholinyl, 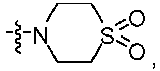 1 H-imidazolyl, 4-methyl-1 H-imidazolyl, piperazinyl, 4-methylpiperazinyl, 4-dimethylaminopiperazinyl, 4-methylaminopiperazinyl, 4-hydroxyethylpiperazinyl, 4-dimethylaminoethylpiperazinyl, piperidinyl, 4-dimethylaminopiperidinyl, pyrrolidinyl, 3-aminopyrrolidinyl, (R)-3-aminopyrrolidinyl, (S)-3-aminopyrrolidinyl, 3-dimethylaminopyrrolidinyl, (R)-3-dimethylaminopyrrolidinyl, (S)-3-dimethylaminopyrrolidinyl, 3-methylaminomethylpyrrolidinyl and 3-amino-3-methylpyrrolidinyl.

Preferably, R₁ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN; further preferably, R₁ is selected from H, C₁₋₅ alkyl, C₁₋₅ alkoxy, halo-substituted C₁₋₅ alkyl, halo-substituted C₁₋₅ alkoxy, -OH, -NH₂, halogen and -CN; and more preferably, R₁ is selected from H, C₁₋₃ alkyl, C₁₋₃ alkoxy, halo-substituted C₁₋₃ alkyl, halo-substituted C₁₋₃ alkoxy, -OH, -NH₂, halogen and CN; wherein the halogen is preferably fluorine or chlorine;

Preferably, R₂ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN; further preferably, R₂ is selected from H, C₁₋₅ alkyl, C₁₋₅ alkoxy, halo-substituted C₁₋₅ alkyl, halo-substituted C₁₋₅ alkoxy, -OH, -NH₂, halogen and -CN; and more preferably, R₂ is selected from H, C₁₋₃ alkyl, C₁₋₃ alkoxy, halo-substituted C₁₋₃ alkyl, halo-substituted C₁₋₃ alkoxy, -OH, -NH₂, halogen and -CN; wherein the halogen is preferably fluorine or chlorine; and
B is selected from the following structures: 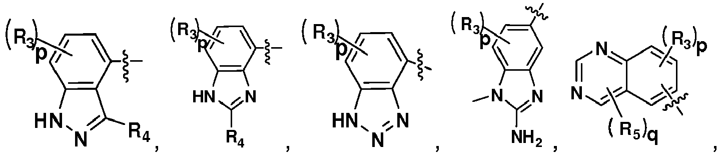 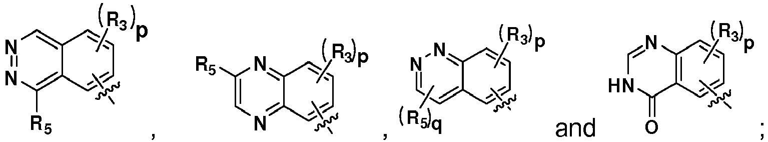 wherein R₃ is preferably selected from H, C₁₋₆ alkyl, C₁₋₆ alkoxy, halo-substituted C₁₋₆ alkyl, halo-substituted C₁₋₆ alkoxy, -OH, -NH_{2,} halogen and -CN; further preferably, R₃ is selected from H, C₁₋₅ alkyl, C₁₋₅ alkoxy, halo-substituted C₁₋₅ alkyl, halo-substituted C₁₋₅ alkoxy, - OH, -NH₂, halogen and -CN; and more preferably, R₃ is selected from H, C₁₋₃ alkyl, C₁₋₃ alkoxy, halo-substituted C₁₋₃ alkyl, halo-substituted C₁₋₃ alkoxy, -OH, -NH₂, halogen and - CN; wherein the halogen(s) is(are) preferably fluorine or chlorine;

R₄ is -NH₂;

R₅ is selected from H, -NR₆R₇, -NHCOR₈, -SO₂R₈, C₁₋₆ alkyl, C₁₋₆ alkoxy, halo-substituted C₁₋₆ alkyl, halo-substituted C₁₋₆ alkoxy, -OH, -NH₂, halogen, -CN, hydrazino and C₁₋₆ alkyl substituted hydrazino; R₅ is preferably selected from H, -NR₆R₇, -NHCOR₈, -SO₂R₈, C₁₋₅ alkyl, C₁₋₅ alkoxy, halo-substituted C₁₋₅ alkyl, halo-substituted C₁₋₅ alkoxy, -OH, -NH₂, halogen, -CN, hydrazino and C₁₋₅ alkyl-substituted hydrazino; and more preferably, R₅ is selected from H, -NR₆R₇, -NHCOR₈, -SO₂R₈, C₁₋₃ alkyl, C₁₋₃ alkoxy, halo-substituted C₁₋₃ alkyl, halo-substituted C₁₋₃ alkoxy, -OH, -NH₂, halogen, -CN, hydrazino and C₁₋₃ alkyl substituted hydrazino; wherein the halogen(s) is(are) preferably fluorine or chlorine; wherein R₆ and R₇ are preferably independently selected from H, C₁₋₆ alkyl, 4-methylsulphonylanilino and 4-aminosulphonylanilino, further preferably, R₆ and R₇ are independently selected from H, C₁₋₅ alkyl, 4-methylsulphonylanilino and 4-aminosulphonylanilino, and more preferably selected from H, C₁₋₃ alkyl, 4-methylsulphonylanilino and 4-aminosulphonylanilino; R₈ is preferably selected from H and C₁₋₆ alkyl, R₈ is further preferably selected from H and C₁₋₅ alkyl, and more preferably selected from H and C₁₋₃ alkyl;
P is preferably selected from 1, 2 and 3;
q is preferably selected from 1 and 2.

In another embodiment, the present invention provides, but not limited to, the following specific compounds:

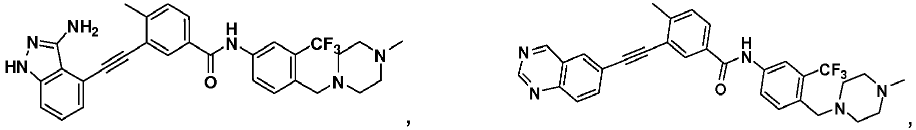

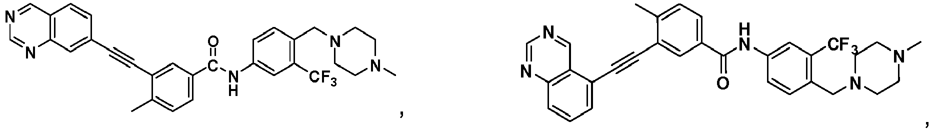

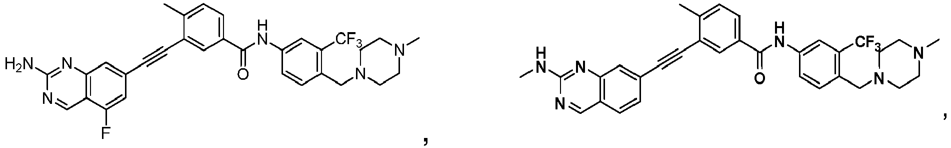

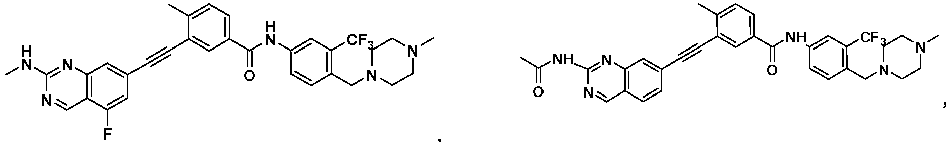

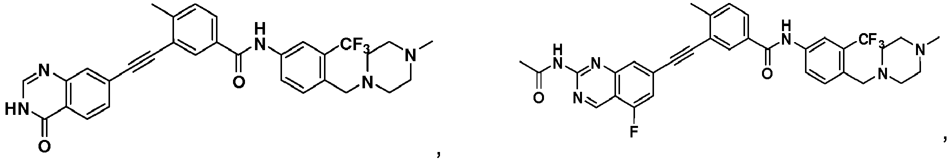

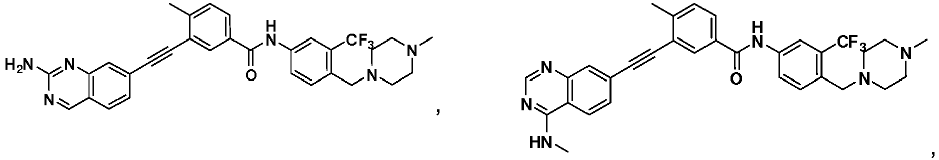

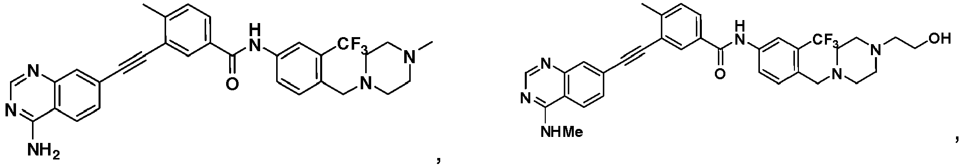

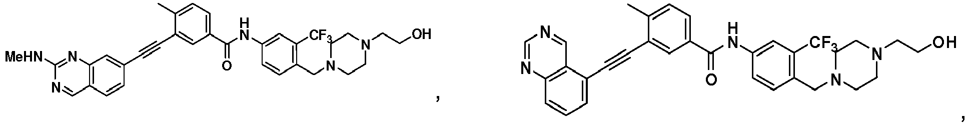

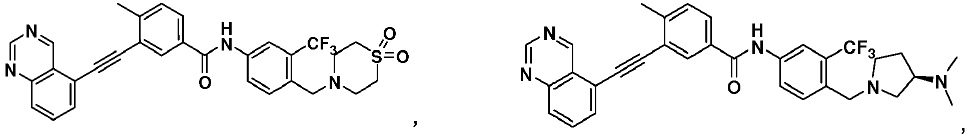

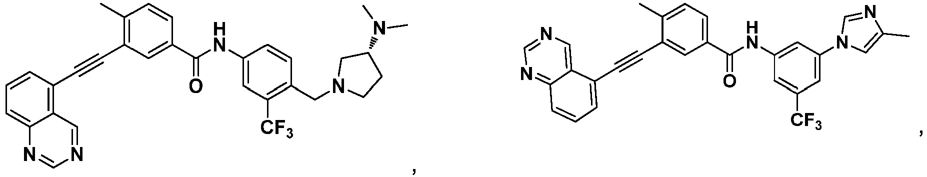

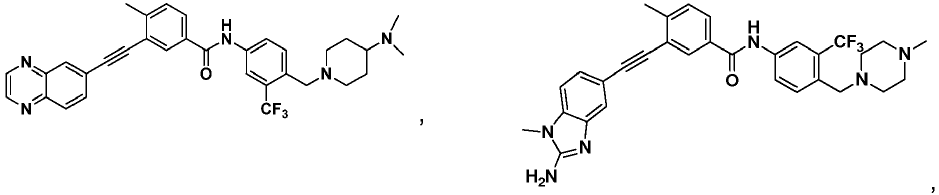

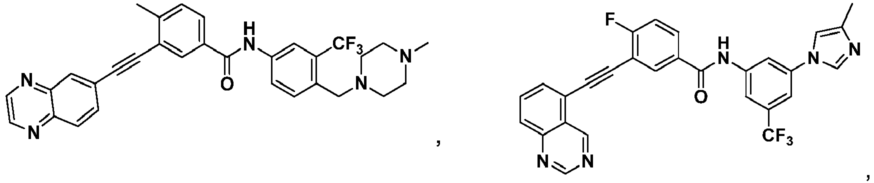

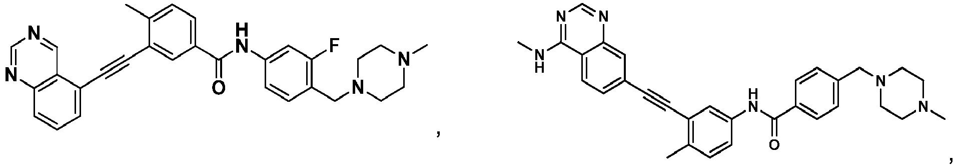

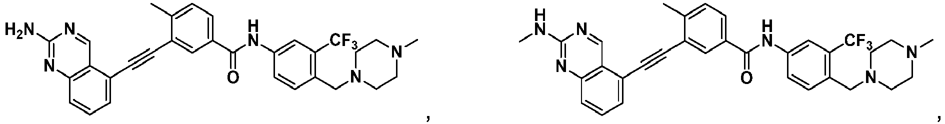

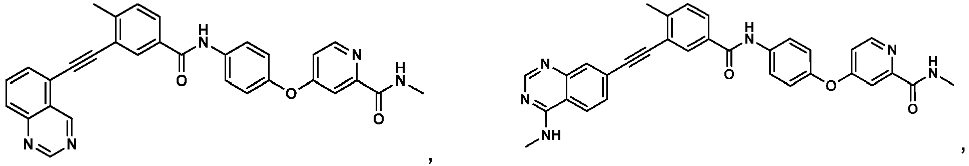

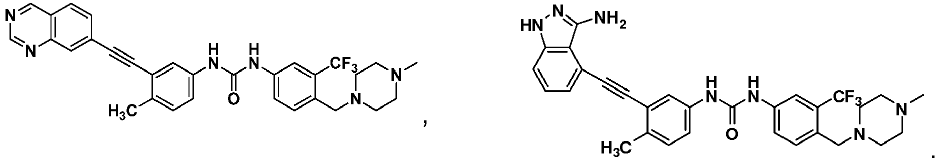

To prepare the compounds of the present invention, the following technical solutions are adopted in the present invention:
Some synthetic routes for preparing the representative intermediates and the final compounds are illustrated as follows:
   1.Synthetic route of the compound of general formula I with L being -C(O)NH-: 2. 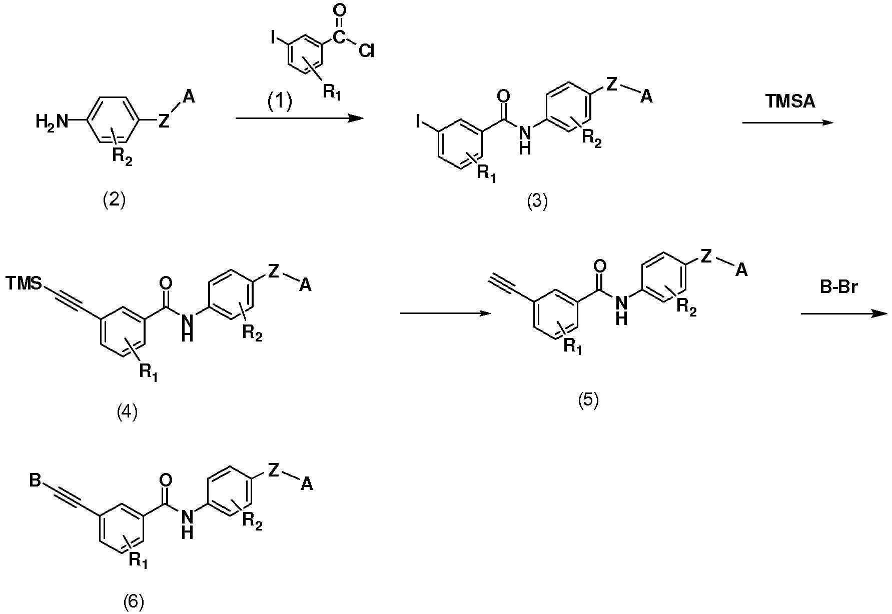 wherein TMSA is trimethylsilylacetylene, and R₁, R₂, Z, A and B are defined as above. The synthesis process is summarized below:

### Step 1: Preparation of Compound (3)

Compound (2) is reacted with Compound (1) at room temperature under alkaline condition, for example, in the presence of triethylamine, to give Compound (3).

### Step 2: Preparation of Compound (4)

Compound (3), Pd(PPh₃)₂Cl₂ and CuI are subjected to Sonogashira reaction with trimethylsilylacetylene under alkaline condition and the protection of an inert gas, to give Compound (4).

### Step 3: Preparation of Compound (5)

The product obtained from Step 2 is deprotected in the presence of potassium carbonate, to give Compound (5).

### Step 4: Preparation of Compound (6)

The product obtained from Step 3, B-Br, Pd(PPh₃)₂Cl₂, CuI and Et₃N are subjected to Sonogashira reaction under the protection of an inert gas, to obtain the title compound, that is, Compound (6).

### 2. Synthetic route of the compound of general formula I with L being -NHC(O)-:

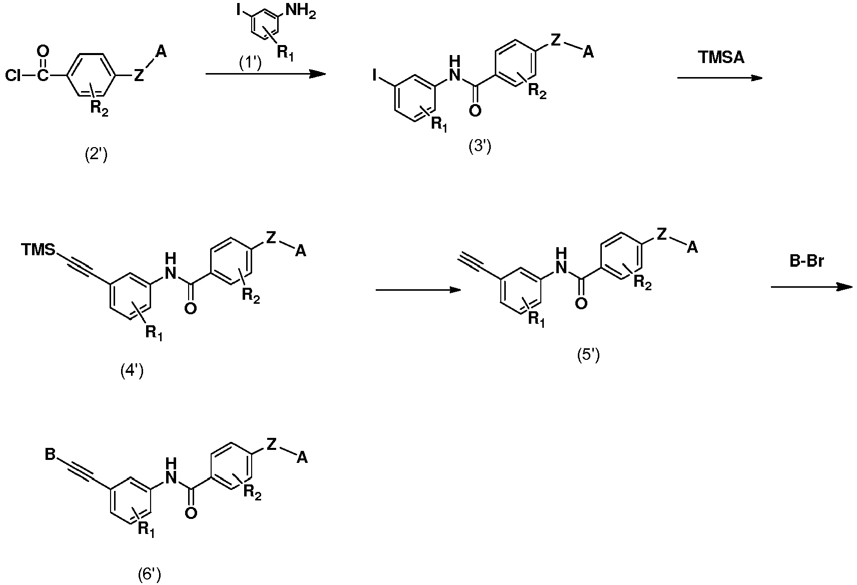

wherein TMSA is trimethylsilylacetylene, and R₁, R₂, Z, A and B are defined as above. The preparation process is the same as the synthetic route of the compound of general formula I with L being -C(O)NH-.

### 3. Synthetic route of the compound of general formula I with L being -NHC(O)NH-:

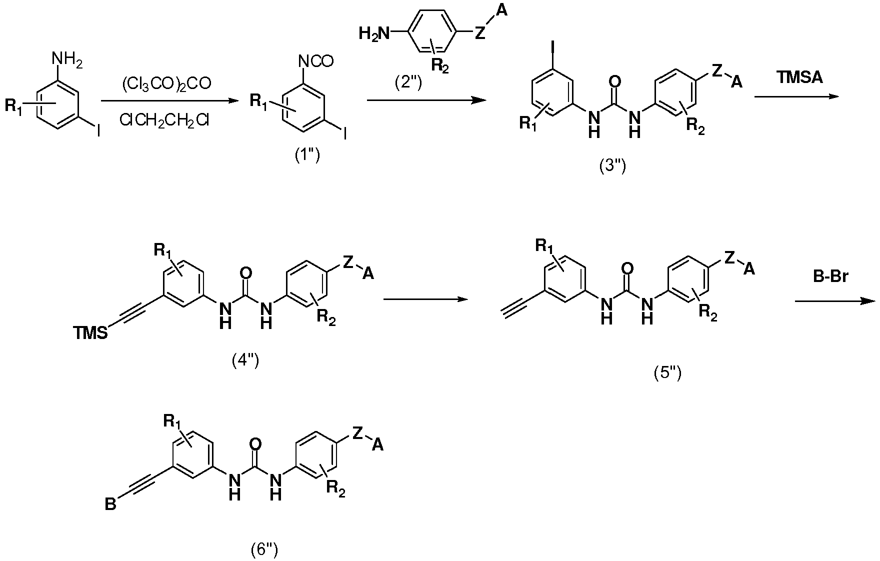

### Step 1: Preparation of Compound (3")

R₁ substituted 3-iodoaniline is reacted with triphosgene and ClCH₂CH₂Cl, to give Compound (1 "). The obtained Compound (1") is reacted with Compound (2") at room temperature under alkaline condition, for example, in the presence of triethylamine, to give Compound (3").

### Step 2: Preparation of Compound (4")

Compound (3"), Pd(PPh₃)₂Cl₂ and CuI are subjected to Sonogashira reaction with trimethylsilylacetylene under alkaline condition and the protection of an inert gas, to give Compound (4").

### Step 3: Preparation of Compound (5")

The product obtained from Step 2 is deprotected in the presence of potassium carbonate, to give Compound (5").

### Step 4: Preparation of Compound (6")

The product obtained from Step 3, B-Br, Pd(PPh₃)₂Cl₂, tricyclohexylphosphine, Cs₂CO₃, DBU (1,8-diazabicyclo[5.4.0]undec-7-ene) and DMF are subjected to Sonogashira reaction under the protection of an inert gas, to obtain the title compound, that is, Compound (6").
wherein TMSA is trimethylsilylacetylene, and R₁, R₂, Z, A and B are defined as above.

### Detailed description of the invention

The following representative embodiments are meant to illustrate the present invention better, but not intended to limit the scope of the invention.

### Example 1: Preparation of 3-(3-amino-1H-indazol-4-yl)ethynyl-4-methyl-N-[4-(4-methylpiperazin-1-yl)methyl-3-trifluoromethylphenyl]benzamide

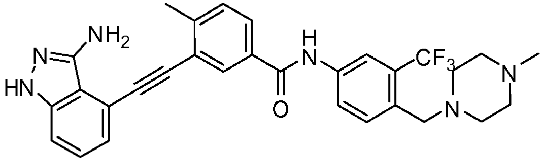

### Step 1: Preparation of 3-iodo-4-methyl-N-[4-(4-methylpiperazin-1-ylmethyl)-3-trifluoromethylphenyl]benzamide

4-(4-methylpiperazin-1-ylmethyl)-3-trifluoromethylaniline (2.27 g, 8.3 mmol), 3-iodo-4-methyl-benzoyl chloride (10 mmol), 15 ml tetrahydrofuran, and 10 ml triethylamine were added into a reactor and stirred at room temperature for 4 hours. After completion of the reaction, the resultant was washed with a saturated NaHCO₃ solution, extracted with ethyl acetate and water, washed with a saturated NaCl solution, dried over anhydrous Na₂SO₄. The solvent was removed by distillation under reduced pressure. The residue was purified by silica gel column chromatography, to give a yellow oily matter.

¹H NMR (500 MHz, CDCl₃) δ: 8.39(s,1 H,N-H), 8.29(s,1 H,Ar-H), 7.88(d, 1 H, Ar-H), 7.86(s, 1 H, Ar-H), 7.75(d, 1 H, Ar-H), 7.73(d, 1 H, Ar-H), 7.28(d, 1 H, Ar-H), 3.62(s, 2H, PhCH₂), 2.60(b, 8H, 4x-CH₂), 2.47(s, 3H,-CH₃), 2.31 (s, 3H,-CH₃).

### Step 2: Preparation of 3-trimethylsilylethynyl-4-methyl-N-[4-((4-methylpiperazin-1-yl)methyl)-3-trifluoromethylphenyl]benzamide

3-iodo-4-methyl-N-[4-(4-methylpiperazin-1-ylmethyl)-3-trifluoromethylphenyl]benzamide (3.1 g, 6.1 mmol), Pd(PPh₃)₂Cl₂ (426 mg, 0.61 mmol) and CuI (231 mg, 1.21 mmol) were added into a reactor, and then 30 ml toluene was added as a solvent. 1 ml triethylamine was added for maintaining an alkaline environment. Under protection of an inert gas, trimethylsilylacetylene (3.0 g, 30.3 mmol) was added to the mixture, and stirred at 58°C for 24 hours. After completion of the reaction, the reaction mixture was extracted with ethyl acetate and water. The organic layers were combined, washed with a saturated NaCl solution, dried over anhydrous Na₂SO₄ and concentrated under reduced pressure. The residue was purified by silica gel column chromatography, to give a yellow solid.

¹H NMR (500 MHz, CDCl₃) δ: 8.30(s, 1H, N-H), 7.86(s, 1H, Ar-H), 7.83(d, 1H, Ar-H), 7.72(s, 1H, Ar-H), 7.55(d, 1H, Ar-H), 7.41 (d, 1H, Ar-H), 7.24(d, 1H, Ar-H), 3.60(s, 2H, PhCH₂), 2.48(b, 8H, 4x-CH₂), 2.45(s, 3H,-CH₃), 2.28(s, 3H,-CH₃), 0.26(s, 9H, 3×-CH₃).

### Step 3: Preparation of 3-ethynyl-4-methyl-N-[4-((4-methylpiperazin-1-yl)methyl)-3-trifluoromethylphenyl]benzamide

The product (1.59 g, 3.3 mmol) obtained from Step 2, potassium carbonate (1.82 g, 13.2 mmol) and 20 ml methanol were mixed in a reactor, and stirred at room temperature for 3 hours under protection of an inert gas. After completion of the reaction, methanol was removed on a rotary evaporator, and the mixture was extracted with ethyl acetate and water. The organic layers were combined, washed with a saturated NaCl solution, and dried over anhydrous Na₂SO₄. The organic solution was concentrated on a rotary evaporator, and the residue was purified by silica gel column chromatography, to give a yellow oily liquid.

¹H NMR (500 MHz, CDCl₃) δ: 10.47(s, 1H, N-H), 8.19(s, 1H, Ar-H), 8.08(s, 1H, Ar-H), 8.04(d, 1 H, Ar-H), 7.91 (d, 1 H, Ar-H), 7.70(d, 1 H, Ar-H), 7.47(d, 1 H, Ar-H), 4.50(s, 1H, =CH), 3.56(s, 2H, PhCH₂), 2.50(s, 3H,-CH₃), 2.36(b, 8H,-4×CH₂), 2.15(s, 3H,-CH₃).

### Step 4: Preparation of 3-((3-amino-1H-indazol-4-yl)ethynyl)-4-methyl-N-[4-((4-methylpiperazin-1-yl)methyl)-3-trifluoromethylphenyl]benzamide

The product (126 mg, 0.3 mmol) obtained from Step 3, 4-bromo-3-amino-1 H-indazole (59 mg, 0.3 mmol), Pd(PPh₃)₂Cl₂ (63 mg, 0.006 mmol), CuI (18 mg, 0.09 mmol), 1 ml Et₃N and 5 ml DMF were added into a 10 ml sealed tube, and reacted with stirring at 80°C for 8 hours under protection of an inert gas. After completion of the reaction, the mixture was extracted with ethyl acetate and water. The organic layers were combined, washed with a saturated NaCl solution, dried over anhydrous Na₂SO₄, and concentrated under reduced pressure. The residue was purified by silica gel column chromatography, to give a white solid.

¹HNMR(300MHz, MeOD)δ: 8.17(d, 1H, J=1.8Hz, Ar-H), 8.14(s, 1H, J=1.8Hz, Ar-H), 8.00(d, 1H, J=9.6Hz, Ar-H), 7.89(q, 1H, J₁=8.1Hz, J₂=1.8Hz, Ar-H), 7.75(d, 1H, J=7.5Hz, Ar-H), 7.47(d, 1H, J=8.1Hz, Ar-H), 7.35(s, 1H, Ar-H), 7.33(d, 1H, J=4.8Hz, Ar-H), 7.23(q, 1 H, J₁=6.0Hz, J₂=1.8Hz, Ar-H), 3.75(s, 2H, PhCH₂), 3.30-2.70(b, 8H, 4x-CH₂), 2.82(s, 3H,-CH₃), 2.64(s, 3H,-CH₃).

ESI-MS *m*/*z:* [M+H]⁺ =547.3 (calculated: 547.2).

### Example 2: Preparation of 3-((quinazolin-6-yl)ethynyl)-4-methyl-N-[4-((4-methylpiperazin-1-yl)methyl)-3-trifluoromethylphenyl]benzamide

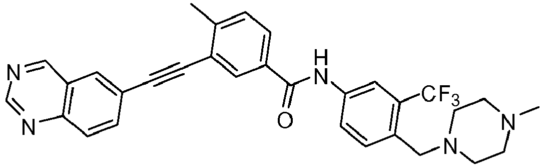

The title compound was prepared using 3-ethynyl-4-methyl-N-[4-((4-methylpiperazin-1-yl)methyl)-3-trifluoromethylphenyl]benzamide and 6-bromoquinazoline according to the method described in Step 4 of Example 1, as an off-white solid.

¹HNMR(500MHz, DMSO-d⁶)δ: 10.54(s, 1H, Ar-H), 9.67(s, 1H, Ar-H), 9.35(s, 1H, Ar-H), 8.47(d, 1H, J=1.5Hz, Ar-H), 8.24(d, 1H, J=1.5Hz, Ar-H), 8.21 (d, 1H, J=1.5Hz, Ar-H), 8.17(q, 1H, J₁=8.5Hz, J₂=1.5Hz, Ar-H), 8.08(d, 1H, J=8.5Hz, Ar-H), 8.06(s, 1H,-NH), 7.96(q, 1H, J₁=8.0Hz, J₂=1.5Hz, Ar-H), 7.71 (d, 1H, J=8.0Hz, Ar-H), 7.56(d, 1H, J=8.0Hz, Ar-H), 3.57(s, 2H, PhCH₂), 2.62(s, 3H,-CH₃), 2.50-2.36(b, 8H, 4x-CH₂), 2.17(s, 3H,-CH₃).

ESI-MS *m*/*z:* [M+H]⁺ =544.3 (calculated: 544.2).

### Example 3: Preparation of 3-((quinazolin-7-yl)ethynyl)4-methyl-N-[4-((4-methylpiperazin-1-yl)methyl)-3-trifluoromethylphenyl]benzamide

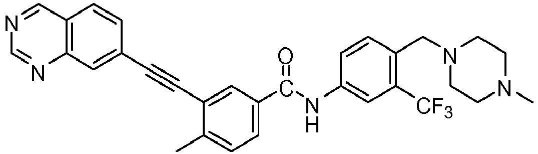

The title compound was prepared using 3-ethynyl-4-methyl-N-[4-((4-methylpiperazin-1-yl)methyl)-3-trifluoromethylphenyl]benzamide and 7-bromoquinazoline according to the method described in Step 4 of Example 1, as a white solid.

¹HNMR (500 MHz, CDCl₃) δ: 9.40 (s, 1 H, Ar-*H*), 9.36 (s, 1H,-NH), 8.20 (s, 1 H, Ar-*H*), 8.14 (s, 1 H, Ar-*H*), 8.07 (s, 1 H, Ar-*H*), 7.93 (s, 1 H, Ar-*H*), 7.92 (s, 1 H, Ar-*H*), 7.89 (s, 1 H, Ar-*H*), 7.81-7.84 (m, 1 H, Ar-*H*), 7.76 (s, 1 H, Ar-*H*), 7.74 (s, 1 H, Ar-*H*), 7.39-7.41 (m, 1 H, Ar-*H*), 3.66 (s, 2H, PhCH₂), 2.63 (s, 3H,-CH₃), 2.58 (b, 8H, 4×-CH₂), 2.39 (s, 3H,-CH₃).

ESI-MS *m*/*z*: [M+H]⁺ = 544.3 (calculated: 544.2).

### Example 4: Preparation of 3-((quinazolin-5-yl)ethynyl)-4-methyl-N-[4-((4-methylpiperazin-1-yl)methyl)-3-trifluoromethylphenyl]benzamide

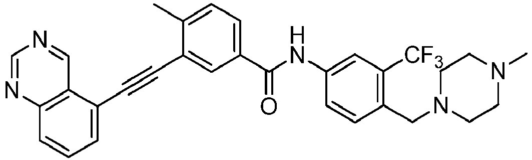

The title compound was prepared using 3-ethynyl-4-methyl-N-[4-((4-methylpiperazin-1-yl)methyl)-3-trifluoromethylphenyl]benzamide and 5-bromoquinazoline according to the method described in Step 4 of Example 1, as a white viscous material.

¹HNMR(300MHz, CDCl₃)δ:9.94(s, 1H, Ar-H), 9.42(s, 1H, Ar-H), 8.13(s, 1H, Ar-H), 8.08(d, 1H, J=7.8Hz, Ar-H), 7.99(s, 1H,-NH), 7.96(d, 1H, J=7.2Hz, Ar-H), 7.95(s, 1H, Ar-H), 7.92(s, 1H, Ar-H), 7.91 (d, 1H, J=4.5Hz, Ar-H), 7.85(q, 1H, J₁=11.1Hz, J₂=1.8Hz, Ar-H), 7.80(d, 1 H, J=8.4Hz, Ar-H), 7.46(d, 1 H, J=8.1 Hz, Ar-H), 3.67(s, 2H, PhCH₂), 2.70(s, 3H,-CH₃), 2.70-2.40(b, 8H, 4x-CH₂), 2.36(s, 3H,-CH₃).

ESI-MS *m*/*z:* [M+H]⁺ =544.3 (calculated: 544.2).

### Example 5: Preparation of 3-((5-fluoro-2-aminoquinazolin-7-yl)ethynyl)-4-methyl-N-[4-((4-methylpiperazin-1-yl)methyl)-3-trifluoromethylphenyl]benzamide

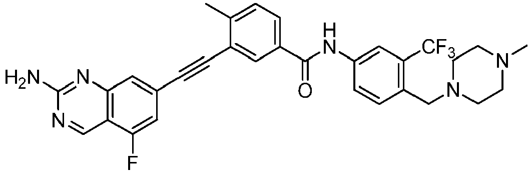

The title compound was prepared using 3-ethynyl-4-methyl-N-[4-((4-methylpiperazin-1-yl)methyl)-3-trifluoromethylphenyl]benzamide and 2-amino-5-fluoro-7-bromoquinazoline according to the method described in Step 4 of Example 1.

¹HNMR(500MHz, CDCl₃) δ:9.28 (s,1H,-NH), 8.08(d,1H,J=1.8,Ar-H), 7.91(s,1H,Ar-H), 7.89(d,1H,J=8.3Hz,Ar-H), 7.86(s, 1H, Ar-H), 7.81 (q, 1H, J₁=8.0Hz, J₂=1.9Hz, Ar-H), 7.78(d, 1 H, J=8.6Hz, Ar-H), 7.54(s, 1 H, Ar-H), 7.40(d, 1 H, J=8.1 Hz, Ar-H), 7.01 (d, 1 H, J=9.7Hz, Ar-H), 5.41 (d, 2H,-NH₂), 3.63(s,2H, PhCH₂), 2.60(s,3H,-CH₃), 2.54-2.48(b,8H, 4x-CH₂), 2.33(s, 3H,-CH₃).

ESI-MS *m*/*z*: [M+H]⁺ =577.3 (calculated: 577.2).

### Example 6: Preparation of 3-((2-methylaminoquinazolin-7-yl)ethynyl)-4-methyl-N-[4-((4-methylpiperazin-1-yl)methyl)-3-trifluoromethylphenyl]benzamide

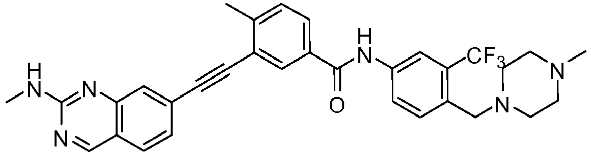

The title compound was prepared using 3-ethynyl-4-methyl-N-[4-((4-methylpiperazin-1-yl)methyl)-3-trifluoromethylphenyl]benzamide and 2-methylamino-7-bromoquinazoline according to the method described in Step 4 of Example 1.

¹HNMR(500MHz, DMSO-d⁶)δ: 8.95(s, 1H,-NH), 8.02(s, 1H, Ar-H), 7.93(d, 1H, J=8.5Hz, Ar-H), 7.92(s, 1 H, Ar-H), 7.87(s, 1 H, Ar-H), 7.82(s, 1 H, Ar-H), 7.80(d, 1 H, J=8.1 Hz, Ar-H), 7.73-7.66(bs, 1H,-NH), 7.64(d, 1H, J=8.2Hz, Ar-H), 7.40(d, 1H, J=8.0Hz, Ar-H), 7.33(d, 1H, J=9.0Hz, Ar-H), 5.30(s, 1H,-NH), 3.71 (s, 2H, PhCH₂), 3.13(d, 3H, J=5.0Hz,-CH₃), 2.90-2.60(b, 8H, 4x-CH₂), 2.62(s, 3H,-CH₃), 2.54(s, 3H,-CH₃).

ESI-MS *m*/*z*: [M+H]⁺ =573.3 (calculated: 573.3).

### Example 7: Preparation of 3-((5-fluoro-2-methylaminoquinazolin-7-yl)ethynyl)-4-methyl-N-[4-((4-methylpiperazin-1-yl)methyl)-3-trifluoromethylphenyl]benzamide

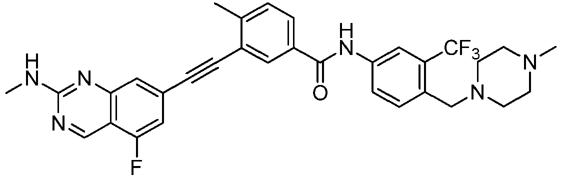

The title compound was prepared using 3-ethynyl-4-methyl-N-[4-((4-methylpiperazin-1-yl)methyl)-3-trifluoromethylphenyl]benzamide and 2-methylamino-5-fluoro-7-bromoquinazoline according to the method described in Step 4 of Example 1.

ESI-MS *m*/*z*: [M+H]⁺ = 591.3 (calculated: 591.2)

### Example 8: Preparation of 3-((2-acetylaminoquinazolin-7-yl)ethynyl)-4-methyl-N-[4-((4-methylpiperazin-1-yl)methyl)-3-trifluoromethylphenyl]benzamide

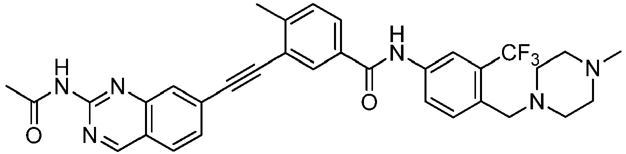

The title compound was prepared using 3-ethynyl-4-methyl-N-[4-((4-methylpiperazin-1-yl)methyl)-3-trifluoromethylphenyl]benzamide and 2-acetylamino-7-bromoquinazoline according to the method described in Step 4 of Example 1.

¹HNMR(500MHz, DMSO-d⁶)δ:10.74(s, 1H,-NH), 10.56(s, 1H,-NH), 9.52(s, 1H, Ar-H), 8.24(s, 1 H, Ar-H), 8.22(s, 1 H, Ar-H), 8.13(d, 1 H, J=8.5Hz, Ar-H), 8.11 (d, 1 H, J=9.5Hz, Ar-H), 7.99(s, 1 H, Ar-H), 7.97(d, 1 H, J=12.0Hz, Ar-H), 7.73(d, 1 H, J=8.0Hz, Ar-H), 7.71 (d, 1 H, J=8.0Hz, Ar-H), 7.56(d, 1 H, J=8.0Hz, Ar-H), 3.65(s, 2H, PhCH₂), 2.62(s, 3H,-CH₃), 2, 50(s, 3H,-CH₃), 2.70-2.30(b, 8H, 4x-CH₂), 2.30(s, 3H,-CH₃).

ESI-MS *m*/*z*: [M+H]⁺ =601.3 (calculated: 601.2).

### Example 9: Preparation of 3-((4-oxo-3,4-dihydroquinazolin-7-yl)ethynyl)-4-methyl-N-[4-((4-methylpiperazin-1-yl)methyl)-3-trifluoromethylphenyl]benzamide

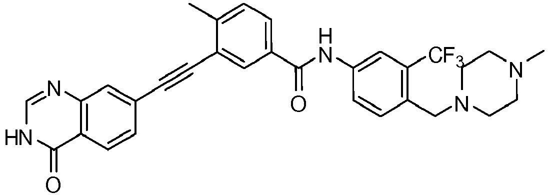

The title compound was prepared using 3-ethynyl-4-methyl-N-[4-((4-methylpiperazin-1-**yl)methyl)-3-trifluoromethylphenyl]benzamide** and 7-bromoquinazolin-4(3H)-one according to the method described in Step 4 of Example 1.

¹HNMR(500MHz, DMSO-d⁶)δ: 10.53(s, 1H,-NH), 8.22(d, 1H, J=5.3Hz, Ar-H), 8.21(d, 1H, J=5.6Hz, Ar-H), 8.14(s, 1 H, Ar-H), 8.13(d, 1 H, J=8.4Hz, Ar-H), 8.07(d, 1 H, J=8.4Hz, Ar-H), 7.94(q, 1H, J₁=8.0Hz, J₂=1.9Hz, Ar-H), 7.80(d, 1H, J=1.4Hz, Ar-H), 7.71 (d, 1H, J=7.7Hz, Ar-H), 7.61 (q, 1H, J₁=8.2Hz, J₂=1.5Hz, Ar-H), 7.54(d, 1H, J=8.2Hz, Ar-H), 3.57(s, 2H, PhCH₂), 2.59(s, 3H,-CH₃), 2.45-2.30(b, 8H, 4x-CH₂), 2.16(s, 3H,-CH₃).

ESI-MS *m*/*z*: [M+H]⁺ =560.2 (calculated: 560.2).

### Example 10: Preparation of 3-((5-fluoro-2-acetylaminoquinazolin-7-yl)ethynyl)-4-methyl-N-[4-((4-methylpiperazin-1-yl)methyl)-3-trifluoromethylphenyl]benzamide

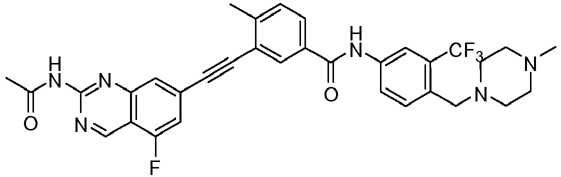

The title compound was prepared using 3-ethynyl-4-methyl-N-[4-((4-methylpiperazin-1-yl)methyl)-3-trifluoromethylphenyl]benzamide and 2-acetylamino-5-fluoro-7-bromoquinazoline according to the method described in Step 4 of Example 1.

¹HNMR(300MHz,DMSC)-d⁶)δ:10.92(s,1H,-NH),10.56(s,1H,-NH),9.63(s,1H,Ar-H),8.22(d,1H,J=9.0Hz,Ar-H),8.22(d,1H,J=9.0Hz,Ar-H),8.06(d,1H,J=8.7Hz,Ar-H),7.98(d,1H,J=6.8Hz,Ar-H),7.84(s,1H,Ar-H),7.72(d,1H,J=8.8Hz,Ar-H),7.60(d,1 H,J=10.7Hz,Ar-H),7.56(d,1 H,J=8.8Hz,Ar-H),3.57(s,2H,PhCH₂),2.61 (s,3H,-CH₃),2.50-2.20(b,8H, 4×-CH₂),2.30(s,3H,-CH₃),2.18(s,3H,-CH₃).

ESI-MS *mlz:* [M+H]⁺ =619.3 (calculated: 619.2).

### Example 11: Preparation of 3-((2-aminoquinazolin-7-yl)ethynyl)-4-methyl-N-[4-((4-methylpiperazin-1-yl)methyl)-3-trifluoromethylphenyl]benzamide

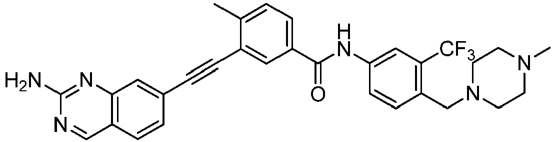

The title compound was prepared using 3-ethynyl-4-methyl-N-[4-((4-methylpiperazin-1-yl)methyl)-3-trifluoromethylphenyl]benzamide and 2-amino-7-bromoquinazoline according to the method described in Step 4 of Example 1, as a white viscous solid.

¹HNMR(500MHz, CDCl₃)δ:9.02(s, 1H, Ar-H), 8.04(d, 1H, J=1.5Hz, Ar-H), 7.98(s, 1H, Ar-H), 7.92(d, 1 H, J=8.5Hz, Ar-H), 7.88(s, 1 H, Ar-H), 7.81 (q, 1 H, J₁=6.5Hz, J₂=1.8Hz, Ar-H), 7.76(s, 1 H, Ar-H), 7.71 (d, 1 H, J=8.5Hz, Ar-H), 7.69(d, 1 H, J=8.5Hz, Ar-H), 7.40(q, 1 H, J₁=8.5Hz, J₂=1.8Hz, Ar-H), 7.39(q, 1H, J₁=8.5Hz, J₂=1.5Hz, Ar-H), 5.26(s, 2H,-NH₂), 3.69(s, 2H, PhCH₂), 2.90-2.60(b, 8H, 4×-CH₂), 2.62(s, 3H,-CH₃), 2.48(s, 3H,-CH₃).

ESI-MS m/z: [M+H]⁺ =559.3 (calculated: 559.2).

### Example 12: Preparation of 3-((4-methylaminoquinazolin-7-yl)ethynyl)-4-methyl-N-[4-((4-methylpiperazin-1-yl)methyl)-3-trifluoromethylphenyl]benzamide

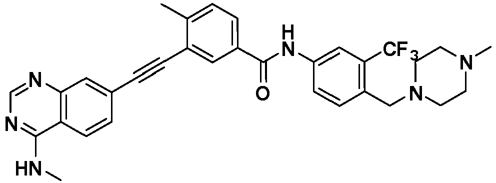

The title compound was prepared using 3-ethynyl-4-methyl-N-[4-((4-methylpiperazin-1-yl)methyl)-3-trifluoromethylphenyl]benzamide and 4-methylamino-7-bromoquinazoline according to the method described in Step 4 of Example 1.

¹HNMR(500MHz, DMSO-d⁶)δ:10.53(s, 1H,-NH), 8.51 (s, 1H, Ar-H), 8.42(d, 1H, J=4.4Hz, Ar-H), 8.24(d, 1H, J=8.6Hz, Ar-H), 8.22(d, 1H, J=1.6Hz, Ar-H), 8.21 (s, 1H, Ar-H), 8.06(d, 1 H, J=8.2Hz, Ar-H), 7.95(d, 1 H, J=8.0Hz, Ar-H), 7.87(s, 1H,-NH), 7.71 (d, 1 H, J=8.4Hz, Ar-H), 7.68(d, 1 H, J=8.5Hz, Ar-H), 7.54(d, 1 H, J=8.2Hz, Ar-H), 3.57(s, 2H, PhCH₂), 3.02(d, 3H, J=4.4Hz,-CH₃), 2.60(s, 3H,-CH₃), 2.40(b, 4H, 2×-CH₂), 2.36(b, 4H, 2×-CH₂), 2.17(s, 3H,-CH₃).

ESI-MS m/z: [M+H]⁺ =573.3 (calculated: 573.3).

### Example 13: Preparation of 3-((4-aminoquinazolin-7-yl)ethynyl)-4-methyl-N-[4-((4-methylpiperazin-1-yl)methyl)-3-trifluoromethylphenyl]benzamide

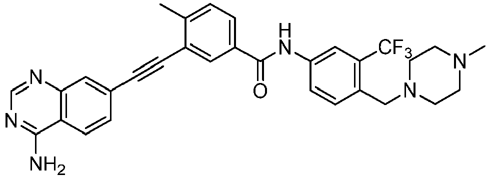

The title compound was prepared using 3-ethynyl-4-methyl-N-[4-((4-methylpiperazin-1-yl)methyl)-3-trifluoromethylphenyl]benzamide and 4-amino-7-bromoquinazoline according to the method described in Step 4 of Example 1.

¹HNMR(500MHz, DMSO-d⁶)δ:10.58(s, 1H, Ar-H), 9.93(s, 1H,-NH), 8.44(s, 1H, Ar-H), 8.29(d, 1 H, J=8.6Hz, Ar-H), 8.23(s, 1 H, Ar-H), 8.23(s, 1 H, Ar-H), 8.12(d, 1 H, J=8.5Hz, Ar-H), 7.96(d, 2H, J=8.0Hz, 2×-NH₂), 7.86(s, 1H, Ar-H), 7.71 (d, 1H, J=8.6Hz, Ar-H), 7.66(d, 1 H, J=8.6Hz, Ar-H), 7.55(d, 1 H, J=8.2Hz, Ar-H), 3.67(s, 2H, PhCH₂), 3.12-2.80(b, 8H, 4x-CH₂), 2.75(s, 3H,-CH₂), 2.60(s, 3H,-CH₃).

ESI-MS *m*/*z:* [M+H]⁺ =559.3 (calculated: 559.2).

### Example 14: Preparation of 3-((4-methylaminoquinazolin-7-yl)ethynyl)-4-methyl-N-[4-((4-(2-hydroxyethyl)piperazin-1-yl)methyl)-3-trifluoromethylphenyl]benzamide

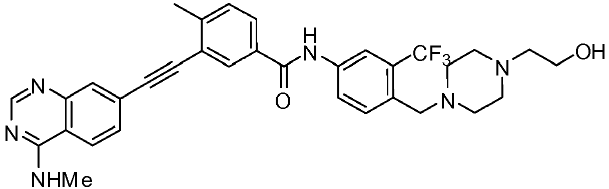

First, 3-ethynyl-4-methyl-N-[4-((4-(2-hydroxyethyl)piperazin-1-yl)methyl)-3-trifluoromethylphenyl]benzamide was prepared according to the method described in Steps 1 to 3 of Example 1.

Then, the title compound was prepared using 3-ethynyl-4-methyl-N-[4-((4-(2-hydroxyethyl)piperazin-1-yl)methyl)-3-trifluoromethylphenyl]benzamide and 4-methylamino-7-bromoquinazoline according to the method described in Step 4 of Example 1.

¹HNMR(500MHz, DMSO-d⁶)δ:10.53(s, 1 H, Ar-H), 8.52(s, 1 H, Ar-H), 8.42(d, 1 H, J=4.5Hz,-NH), 8.24(d, 1H, J=8.5Hz, Ar-H), 8.22(d, 1H, J=1.5Hz, Ar-H), 8.21 (s, 1H, Ar-H), 8.07(d, 1H, J=8.0Hz, Ar-H), 7.95(q, 1H, J₁=8.0Hz, J₂=1.5Hz, Ar-H), 7.87(s, 1H,-NH), 7.72(d, 1H, J=8.5Hz, Ar-H), 7.68(q, 1H, J₁= 8.5Hz, J₂=1.5Hz, Ar-H), 7.54(d, 1H, J=8.5Hz, Ar-H), 4.34(s, 1H, -OH), 3.58(b, 2H,-CH₂), 3.50(s, 2H, PhCH₂), 3.02(d, 3H,-CH₃), 2.60(s, 3H,-CH₃), 2.50(b, 2H,-CH₂), 2.50-2.30(b, 8H,-CH₂).

ESI-MS m/z: [M+H]⁺ =603.3 (calculated: 603.2).

### Example 15: Preparation of 3-((2-methylaminoquinazolin-7-yl)ethynyl)-4-methyl-N-[4-((4-(2-hydroxyethyl)piperazin-1-yl)methyl)-3-trifluoromethylphenyl]benzamide

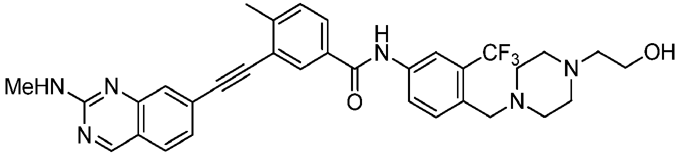

According to the method described in Example 14, the title compound was prepared using 3-ethynyl-4-methyl-N-[4-((4-(2-hydroxyethyl)piperazin-1-yl)methyl)-3-trifluoromethylphenyl]benzamide and 2-methylamino-7-bromoquinazoline.

¹HNMR(500MHz, CDCl₃)δ:8.95(s, 1H,-NH), 8.02(d, 1H, J=1.6Hz, Ar-H), 7.91 (d, 1H, J=9.4Hz, Ar-H), 7.90(s, 1H, Ar-H), 7.87(s, 1H, Ar-H), 7.82(s, 1H, Ar-H), 7.80(q, 1H, J₁=8.0Hz, J₂=1.8Hz, Ar-H), 7.75(d, 1 H, J=8.5Hz, Ar-H), 7.64(d, 1 H, J=8.2Hz, Ar-H), 7.40(d, 1H, J=8.0Hz, Ar-H), 7.33(q, 1H, J₁=8.2Hz, J₂=1.2Hz, Ar-H), 5.30(m, 1H, -OH), 3.67-3.70(m, 4H, 2×-CH₂), 3.13(d, 3H, J=5.1Hz,-CH₃), 2.62-2.67(m, 10H, 5×-CH₂), 2.62(s, 3H,-CH₃).

ESI-MS m/z: [M+H]⁺ =603.3 (calculated: 603.2).

### Example 16: Preparation of 3-((quinazolin-5-yl)ethynyl)-4-methyl-N-[4-((4-(2-hydroxyethyl)piperazin-1-yl)methyl)-3-trifluoromethylphenyl]benzamide

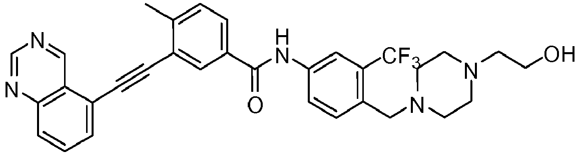

According to the method described in Example 14, the title compound was prepared using 3-ethynyl-4-methyl-N-[4-((4-(2-hydroxyethyl)piperazin-1-yl)methyl)-3-trifluoromethylphenyl]benzamide and 5-bromoquinazoline.

¹HNMR(500MHz, CDCl₃)δ:9.92(s, 1H, Ar-H), 9.40(s, 1H, Ar-H), 8.12(s, 1H, Ar-H), 8.07(s, 1 H, -NH), 8.06(d, 1 H, J=8.3Hz, Ar-H), 7.92(d, 1 H, J=7.2Hz, Ar-H), 7.92(d, 1 H, J=7.2Hz, Ar-H), 7.89(d, 1H, J=9.5Hz, Ar-H), 7.89(s, 1H, Ar-H), 7.84(q, 1H, J₁=8.0Hz, J₂=1.8Hz, Ar-H), 7.78(d, 1 H, J=8.4Hz, Ar-H), 7.43(d, 1 H, J=8.0Hz, Ar-H), 5.30(s, 1 H, -OH), 3.65(s, 2H, PhCH₂), 3.63(t, 2H, J=5.2Hz,-CH₂), 2.68(s, 3H,-CH₃), 2.58(t, 2H, J=5.0Hz,-CH₂), 2.65-2.50(b, 8H,-CH₂).

ESI-MS m/z: [M+H]⁺ =574.3 (calculated: 574.2).

### Example 17: Preparation of 3-((quinazolin-5-yl)ethynyl)-4-methyl-N-(4-thiomorpholinyl-1,1-dioxo-3-trifluoromethylphenyl)benzamide

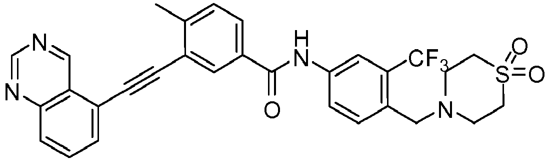

First, 3-ethynyl-4-methyl-N-(4-thiomorpholinyl-1,1-dioxo-3-trifluoromethylphenyl)benzamide was prepared according to the method described in Steps 1 to 3 of Example 1.

Then, the title compound was prepared using 3-ethynyl-4-methyl-N-(4-thiomorpholinyl-1,1-dioxo-3-trifluoromethylphenyl)benzamide and 5-bromoquinazoline according to the method described in Step 4 of Example 1.

¹HNMR(500MHz, DMSO) δ(ppm): 10.58(s, 1H,-NH), 9.95(s, 1 H, Ar-H), 9.43(s, 1 H, Ar-H), 8.37(d, 1 H, Ar-H), 8.24(d, 1 H, Ar-H), 8.07-8.12(m, 4H, Ar-H), 7.98(dd, 1 H, Ar-H), 7.80(d, 1 H, Ar-H), 7.58(d, 1 H, Ar-H), 3.78(s, 2H, PhCH₂), 3.13(d, 4H,-CH₃), 2.906(d, 4H,-CH₃), 2.68(s, 3H,-CH₃).

ESI-MS m/z: [M+H]⁺ =579.2 (calculated: 579.1).

### Example 18: Preparation of 3-((quinazolin-5-yl)ethynyl)-4-methyl-(R)-N-[4-((3-(dimethylamino)pyrrolidin-1-yl)methyl)-3-trifluoromethylphenyl]benzamide

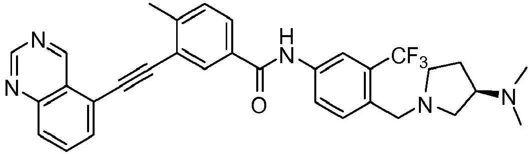

3-ethynyl-4-methyl-(R)-N-[4-((3-(dimethylamino)pyrrolidin-1-yl)methyl)-3-trifluoromethylphenyl]benzamide was prepared according to the method described in Steps 1 to 3 of Example 1.

The title compound was prepared using 3-ethynyl-4-methyl-(R)-N-[4-((3-(dimethylamino)pyrrolidin-1-yl)methyl)-3-trifluoromethylphenyl]benzamide and 5-bromoquinazoline according to the method described in Step 4 of Example 1.

¹HNMR (500MHz, DMSO) δ(ppm): 10.55(s, 1H,-NH), 9.95(s, 1 H, Ar-H), 9.43(s, 1 H, Ar-H), 8.37(d, 1 H, Ar-H), 8.27(m, 1 H, Ar-H), 8.06-8.12(m, 4H, Ar-H), 7.99(d, 1 H, Ar-H), 7.71 (d, 1 H, Ar-H), 7.56(d, 1 H, Ar-H), 3.69(q, 2H,-CH₂), 2.73-2.76(m, 1H,-CH), 2.68(s, 3H,-CH₃), 2.64-2.68(m, 1H,-CH), 2.57-2.62(m, 1H,-CH), 2.34-2.67(m, 1H,-CH), 2.10(s, 6H,-CH₃), 1.84-1.89(m, H,-CH), 1.75-1.77(m, 1H,-CH₂-), 1.63-1.66(m, 1H,-CH).

ESI-MS m/z: [M+H]⁺ =558.3 (calculated: 558.2).

### Example 19: Preparation of 3-((quinazolin-5-yl)ethynyl)-4-methyl-(S)-N-[4-((3-(dimethylamino)pyrrolidin-1-yl)methyl)-3-trifluoromethylphenyl]benzamide

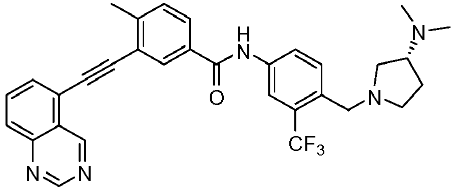

3-ethynyl-4-methyl-(S)-N-[4-((3-(dimethylamino)pyrrolidin-1-yl)methyl)-3-trifluoromethylphenyl]benzamide was prepared according to the method described in Steps 1 to 3 of Example 1.

The title compound was prepared using 3-ethynyl-4-methyl-(S)-N-[4-((3-(dimethylamino)pyrrolidin-1-yl)methyl)-3-trifluoromethylphenyl]benzamide and 5-bromoquinazoline according to the method described in Step 4 of Example 1.

¹HNMR(500MHz, DMSO)δ: 10.56(s, 1H, N-H), 9.95(s, 1H, Ar-H), 9.43(s, 1H, Ar-H), 8.37(d, 1 H, Ar-H), 8.21 (d, 1 H, Ar-H), 8.12(t, 1 H, Ar-H), 8.10(d, 1 H, Ar-H), 8.09(t, 1 H, Ar-H), 8.07(t, 1 H, Ar-H), 7.98(dd, 1 H, Ar-H), 7.71 (d, 1 H, Ar-H), 7.58(d, 1 H, Ar-H), 3.69(dd, 2H,-CH₂), 2.68(s, 3H,-CH₃), 2.64-2.68(m, 2H,-CH), 2.57-2.62(m, 1H,-CH), 2.14(s, 6H,-CH₃), 1.91 (m, 2H,-CH₂-), 1.77(m, 1H,-CH₂-), 1.66(m, 1H,-CH₂-).

ESI-MS m/z: [M+H]⁺ =558.2 (calculated: 558.2).

### Example 20: Preparation of 3-((quinazolin-5-yl)ethynyl)-4-methyl-N-[3-(4-methyl-1H-imidazol-1-yl)-5-trifluoromethylphenyl]benzamide

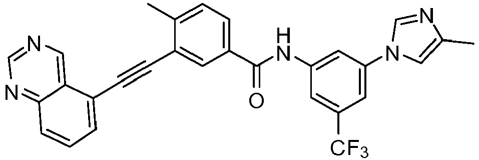

3-ethynyl-4-methyl-N-[3-(4-methyl-1H-imidazol-1-yl)-5-trifluoromethylphenyl]benzamide was prepared according to the method described in Steps 1 to 3 of Example 1.

The title compound was prepared using 3-ethynyl-4-methyl-N-[3-(4-methyl-1H-imidazol-1-yl)-5-trifluoromethylphenyl]benzamide and 5-bromoquinazoline according to the method described in Step 4 of Example 1.

¹HNMR(300MHz, DMSO)δ:10.78(s, 1 H, N-H), 9.94(s, 1 H, Ar-H), 9.43(s, 1 H, Ar-H), 8.40(d, 1 H, Ar-H), 8.34(s, 1 H, Ar-H), 8.21 (s, 1 H, Ar-H), 8.19(t, 1 H, Ar-H), 8.10(d, 1 H, Ar-H), 8.09(s, 1 H, Ar-H), 8.08(t, 1 H, Ar-H), 8.02(dd, 1 H, Ar-H), 7.74(s, 1 H, Ar-H), 7.59(d, 1 H, Ar-H), 7.49(s, 1 H, Ar-H), 2.68(s, 3H,-CH₃), 2.19(s, 3H,-CH₃).

ESI-MS m/z: [M+H]⁺ =512.3 (calculated: 512.2).

### Example 21: Preparation of 3-(quinoxalin-6-yl)ethynyl)-4-methyl-N-[(4-((N,N-dimethylamino)piperidin-1-yl)methyl)-3-trifluoromethylphenyl]benzamide

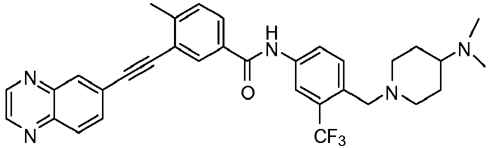

3-ethynyl-4-methyl-N-[(4-((N,N-dimethylamino)piperidin-1-yl)methyl)-3-trifluoromethylphenyl]benzamide was prepared according to the method described in Steps 1 to 3 of Example 1.

The title compound was prepared using 3-ethynyl-4-methyl-N-[(4-((N,N-dimethylamino)piperidin-1-yl)methyl)-3-trifluoromethylphenyl]benzamide and 5-bromoquinoxaline according to the method described in Step 4 of Example 1.

¹HNMR(500MHz, DMSO) δ(ppm): 10.56(s, 1H,-NH), 9.95(s, 1 H, Ar-H), 9.43(s, 1 H, Ar-H), 8.37(d, 1 H, Ar-H), 8.22(d, 1 H, Ar-H), 8.06-8.12(m, 4H, Ar-H), 7.98(dd, 1 H, Ar-H), 7.74(d, 1 H, Ar-H), 7.58(d, 1H,, Ar-H), 3.56(s, 2H, PhCH₂), 2.82(d, 2H,-CH₂), 2.68(s, 3H,-CH₃), 2.17(s, 6H,-CH₃), 2.02-2.14(m, 1H,-CH), 1.99(t, 2H,-CH₂), 1.73(d, 2H,-CH₂), 1.40(m, 2H, CH₂).
ESI-MS m/z: [M+H]⁺ =572.3 (calculated: 572.2).

### Example 22: Preparation of 3-((2-amino-1-methyl-1H-benzo[d]imidazol-5-yl)ethynyl)-4-methyl-N-[(4-(4-methylpiperazin-1-yl)methyl)-3-trifluoromethylphenyl]benzamide

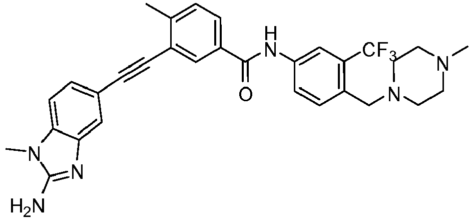

The title compound was prepared using 3-ethynyl-4-methyl-N-[(4-(4-methylpiperazin-1-yl)methyl)-3-trifluoromethylphenyl]benzamide and 5-bromo-2-amino-1-methyl-1 H-benzo[d]imidazole according to the method described in Step 4 of Example 1.

ESI-MS m/z: [M+H]⁺ =561.6 (calculated: 561.2).

### Example 23: Preparation of 3-(quinoxalin-6-yl)ethynyl)-4-methyl-N-[(4-(4-methylpiperazin-1-yl)methyl)-3-trifluoromethylphenyl]benzamide

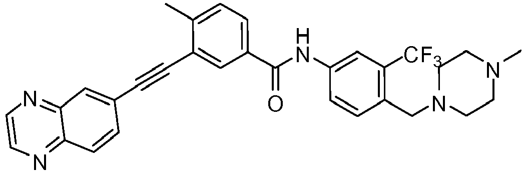

The title compound was prepared using 3-ethynyl-4-methyl-N-[(4-(4-methylpiperazin-1-yl)methyl)-3-trifluoromethylphenyl]benzamide and 6-bromoquinoxaline according to the method described in Step 4 of Example 1.

¹HNMR(500MHz, DMSO) δ(ppm): 10.53(d, 1 H, NH), 9.02(d, 1 H, Ar-H), 8.99(d, 1 H, Ar-H), 8.33(s, 1 H, Ar-H), 8.25(d, 1 H, Ar-H), 8.22(d, 1 H, Ar-H), 8.17(d, 1 H, Ar-H), 8.07(dd, 1 H, Ar-H), 8.02(dd, 1 H, Ar-H), 7.96(dd, 1 H, Ar-H), 7.71 (d, 1 H, Ar-H), 7.55(d, 1 H, Ar-H), 3.57(s, 2H, PhCH₂), 2.62(s, 3H,-CH₃), 2.40(s,4H,-CH₂), 2.34(s,4H,-CH₂), 2.16(s,3H,-CH₃).

ESI-MS m/z: [M+H]⁺ =544.3 (calculated: 544.2).

### Example 24: Preparation of 3-((quinazolin-5-yl)ethynyl)-4-fluoro-N-[3-(4-methyl-1H-imidazol-1-yl)-5-trifluoromethylphenyl]benzamide

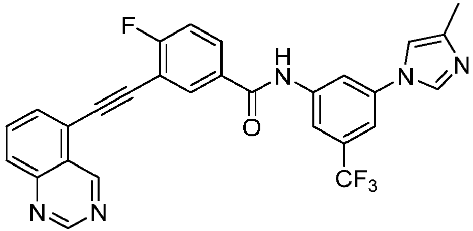

The title compound was prepared using 3-ethynyl-4-fluoro-N-[3-(4-methyl-1H-imidazol-1-yl)-5-trifluoromethylphenyl]benzamide and 5-bromoquinazoline according to the method described in Example 20.

¹HNMR(500MHz, DMSO)δ:10.82(s, 1H, N-H), 9.94(s, 1H, Ar-H), 9.45(s, 1H, Ar-H), 8.53(dd, 1 H, Ar-H), 8.30(s, 1 H, Ar-H), 8.21 (t, 1 H, Ar-H), 8.16(d, 1 H, Ar-H), 8.15(t, 1 H, Ar-H), 8.12(t, 1 H, Ar-H), 8.10(t, 1 H, Ar-H), 8.08(t, 1 H, Ar-H), 7.76(s, 1 H, Ar-H), 7.66(t, 1 H, Ar-H), 7.50(s, 1 H, Ar-H), 2.19(s, 3H,-CH₃).

ESI-MS m/z: [M+H]⁺ =516.1 (calculated: 516.2).

### Example 25: Preparation of 3-((quinazolin-5-yl)ethynyl)-4-methyl-N-[(4-(4-methylpiperazin-1-yl)methyl)-3-fluorophenyl]benzamide

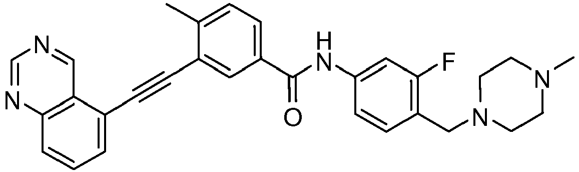

The title compound was prepared using 3-ethynyl-4-methyl-N-[(4-(4-methylpiperazin-1-yl)methyl)-3-fluorophenyl]benzamide and 5-bromoquinazoline according to the method described in Step 4 of Example 1.

¹HNMR(500MHz, DMSO)δ:10.56(s, 1 H,-NH), 9.94(s, 1 H, Ar-H), 9.43(s, 1 H, Ar-H), 8.36(d, 1 H, Ar-H), 8.25(d, 1 H, Ar-H), 8.00-8.11 (m, 3H, Ar-H), 7.52-7.76(m, 2H, Ar-H), 7.32-7.37(m, 1 H, Ar-H), 7.16(m, 1 H, Ar-H), 2.60 (s, 3H,-CH₃), 2.33-2.41 (m, 8H,-CH₂), 2.18 (s, 3H,-CH₃).
ESI-MS m/z: [M+H]⁺ =494.2 (calculated: 494.2).

### Example 26: Preparation of 3-((4-methylaminoquinazolin-7-yl)ethynyl)-4-methyl-N-[(4-(4-methylpiperazin-1-yl)methyl)phenyl]benzamide

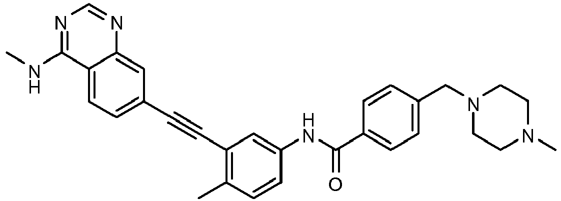

The title compound was prepared using 3-ethynyl-4-methyl-N-[(4-(4-methylpiperazin-1-yl)methyl)phenyl]benzamide and 4-methylamino-7-bromoquinazoline according to the method described in Step 4 of Example 1.

¹HNMR(500MHz,DMSO)δ:10.23(s,1H,N-H),8.50(s,1H,Ar-H),8.40(d,1H,Ar-H),8.22(d,1H,Ar-H),8.05(d,1 H,Ar-H),7.92(d,2H,Ar-H),7.71 (dd,1H,Ar-H),7.66(dd,1H,Ar-H),7.45(d,2H,Ar-H),7.33(d,1H,Ar-H),3.54(s,2H,-CH₂),3.02(d,3H,-CH₃), 2.49 (s,3H,-CH₃), 2.36(m,8H,-CH₂),2.16(s,3H,-CH₃).

ESI-MS m/z: [M+H]⁺ =505.2 (calculated: 505.3).

### Example 27: Preparation of 3-((2-aminoquinazolin-5-yl)ethynyl)-4-methyl-N-[(4-(4-methylpiperazin-1-yl)methyl)-3-trifluoromethylphenyl]benzamide

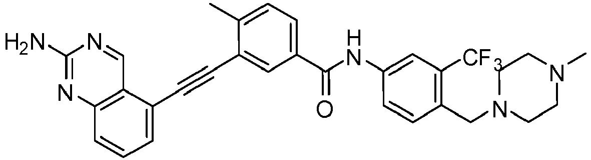

The title compound was prepared using 3-ethynyl-4-methyl-N-[(4-(4-methylpiperazin-1-yl)methyl)-3-trifluoromethylphenyl]benzamide and 2-amino-5-bromoquinazoline according to the method described in Step 4 of Example 1.

¹HNMR(500MHz,DMSO)δ:10.54(s,1H,N-H),9.45(s,1H,Ar-H),8.30(d,1H,Ar-H),8.21 (d,1H,Ar-H),8.07(dd,1 H,Ar-H),7.96(dd,1H,Ar-H),7.71-7.75(m,2H,Ar-H),7.56(d,1H,Ar-H),7.50(t,2H, Ar-H), 7.02(s,2H, N-H),3.57(s,2H,-CH₂),2.64(d,3H,-CH₃),2.38(m,8H,-CH₂), 2.17(s,3H,-CH₃).

ESI-MS m/z: [M+H]⁺ =559.3 (calculated: 559.2).

### Example 28: Preparation of 3-((2-methylaminoquinazolin-5-yl)ethynyl)-4-methyl-N-[(4-((4-methyl)piperazin-1-yl)methyl)-3-trifluoromethylphenyl]benzamide

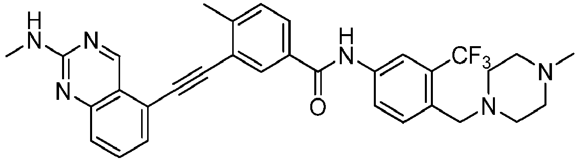

The title compound was prepared using 3-ethynyl-4-methyl-N-[(4-((4-methyl)piperazin-1-yl)methyl)-3-trifluoromethylphenyl]benzamide and 2-methylamino-5-bromoquinazoline according to the method described in Step 4 of Example 1.

¹HNMR(300MHz,DMSO)δ:10.55(s,1H,N-H),9.44(s,1H,N-H),8.31 (d,1H,Ar-H),8.22(d,1H,Ar-H),8.07(dd,1H,Ar-H),7.96(dd,1H,Ar-H),7.75(d,1 H,Ar-H),7.72(d,1 H,Ar-H),7.58(s,1 H,Ar-H),7.55(s,1 H,Ar-H),7.52(d,1H,Ar-H),7.49(d,1H,Ar-H),3.57(s,2H,-CH₂),2.92(d,6H,-CH₃),2.64(s,3H,-CH₃),2.37(m,8H,-CH₂),2.16(s,3H,-CH₃).

ESI-MS m/z: [M+H]⁺ =573.2 (calculated: 573.6).

### Example 29: Preparation of 3-((quinazolin-5-yl)ethynyl)-4-methyl-N-[4-(2-(N-methylcarboxamido)pyridinyl-4-oxy)phenyl]benzamide

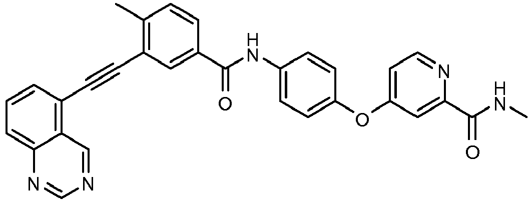

### Step 1: Preparation of 3-iodo-4-methyl-N-N-[4-(2-(N-methylcarboxamido)pyridinyl-4-oxy)phenyl]benzamide

The title compound was prepared using 4-(4-aminophenoxy)-N-methylpicolinamide and 3-iodo-4-methyl-benzoyl chloride according to the method described in Step 1 of Example 1.

### Step 2: Preparation of 3-trimethylsilylethynyl-4-methyl-N-[4-(2-(N-methylcarboxamido)pyridinyl-4-oxy)phenyl]benzamide

The title compound was prepared using the product obtained from Step 1 and 3-trimethylsilylacetylene according to the method described in Step 2 of Example 1.

### Step 3: Preparation of 3-ethynyl-4-methyl-N-[4-(2-(N-methylcarboxamido)pyridinyl-4-oxy)phenyl]benzamide

The title compound was prepared using the product obtained from Step 2 as raw material according to the method described in Step 3 of Example 1.

### Step 4: Preparation of 3-(quinazolin-5-yl)ethynyl)-4-methyl-N-[4-(2-(N-methylcarboxamido)pyridinyl-4-oxy)phenyl]benzamide

The title compound was prepared using the product obtained from Step 3 as raw material according to the method described in Step 4 of Example 1.

¹HNMR(500MHz, DMSO)δ:10.58(s, 1H,-NH), 9.95(s, 1 H, Ar-H), 9.43(s, 1 H, Ar-H), 8.37(d, 1H. Ar-H). 8.36 (d, 1H. Ar-H). 8.24(d. 1H. Ar-H), 8.06-8.11 (m. 3H. Ar-H), 7.99(dd. 1H, Ar-H), 7.68(d, 1 H, Ar-H), 7.46 (d, 2H, Ar-H), 7.042-7.06 (m, 2H, Ar-H), 6.93-6.95(m, 1 H, Ar-H), 6.90(s, 1 H, -NH), 3.00(d, 3H, CH₃).

ESI-MS m/z: [M+H]⁺ =514.2 (calculated: 514.2).

### Example 30: Preparation of 3-((4-methylaminoquinazolin-7-yl)ethynyl)-4-methyl-N-[[4-(2-(N-methylcarboxamido)pyridinyl-4-oxy)phenyl]benzamide

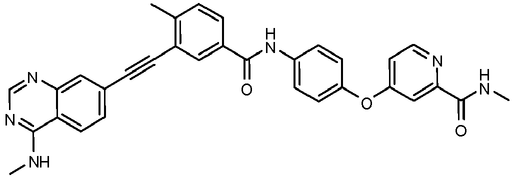

The title compound was prepared using 3-ethynyl-4-methyl-N-[4-(2-(N-methylcarboxamido)pyridinyl-4-oxy)phenyl]benzamide and 4-methylamino-7-bromoquinazoline according to the method described in Example 29.

¹HNMR(500MHz,DMSO)δ:10.44(s,1H,N-H),8.75(d,1H,Ar-H),8.52(s,1H,Ar-H),8.51 (s,1 H,Ar-H),8.25(d,1H,Ar-H),8.21 (s,1H,Ar-H),7.95(d,1H,Ar-H),7.93(d,3H,Ar-H),7.87(s,1H,Ar-H),7.69(d,1H,Ar-H),7.54(d,1H,N-H),7.41 (d,1H,Ar-H),7.24(d,2H,Ar-H),7.17(dd,1H,N-H),3.02(d,3H,-CH₃),2.79(d,3H,-CH₃),2.61 (d,3H,-CH₃).

ESI-MS m/z: [M+H]⁺ =543.3 (calculated: 543.2).

### Example 31: Preparation of N-[3-((quinazolin-7-yl)ethynyl)-4-methyl]phenyl-N'-[4-((4-methylpiperazin-1-yl)methyl)-3-trifluoromethylphenyl)-yl]urea

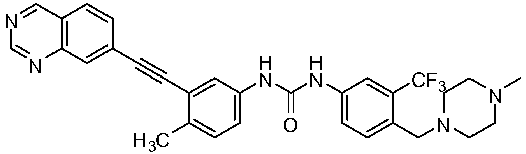

### Step 1: Preparation of N-(3-iodo-4-methylphenyl)-N'-(4-((4-methylpiperazin-1-yl)methyl)-3-trifluoromethylphenyl)urea

Triphosgene (1.04 g, 3.5 mmol) and ClCH₂CH₂Cl (20 mL) were added into a 100 mL round-bottomed flask, and stirred at room temperature until triphosgene was completely dissolved and the system appears colorless and transparent. The reaction system was placed in an ice-salt bath and stirred, 3-iodo-4-methyl aniline (1.64 g, 7 mmol) in ClCH₂CH₂Cl (20 mL) solution was slowly added dropwise, and the system appears yellow milky. After the addition was complete, the mixture was stirred at room temperature for 4 hours. After the reaction was complete by TLC monitoring, Et₃N (1.43 g, 14 mmol) was added, and stirred at room temperature for 0.5 hour. 4-(4-methylpiperazin-1-ylmethyl)-3-trifluoromethylaniline(1.87 g, 7 mmol) was added and stirred at room temperature for 16 hours, and then the starting materials were monitored by TLC and LC-MS until the reaction was complete. The volatiles were removed by distillation under reduced pressure, and the residue was extracted with ethyl acetate (30 ml x 3) and H₂O (30 mL). The organic phases were combined, dried over anhydrous Na₂SO₄, concentrated and purified by column chromatography, to give a yellow solid.

ESI-MS m/z: [M+H]⁺ = 533.2.

### Step 2: Preparation of N-(4-methyl-3-((trimethylsilyl)ethynyl)phenyl)-N'-(4-((4-methylpiperazin-1-yl)methyl)-3-trifluoromethylphenyl)urea

The product (1.06 g, 2.0 mmol) obtained from Step 1, Cul (0.19 g, 0.1 mmol), Pd(PPh₃)Cl₂ (0.35 g, 0.5 mmol) and DMF (10 mL) were added into a 100 mL three-necked flask, and Et₃N (0.52 g, 4.0 mmol) and trimethylsilylacetylene (0.98 g, 10 mmol) were added under protection of an inert gas. The mixture was reacted at 80°C for 16 hours with stirring, and the system was cooled to room temperature, filtered, and extracted with ethyl acetate (50 mL x 3) and H₂O (50 mL). The organic phases were back-extracted with saturated brine, and the organic phases were combined, dried over anhydrous Na₂SO₄, and purified by column chromatography, to give a milky white solid.

### Step 3: Preparation of N-(3-ethynyl-4-methylphenyl)-N'-(4-((4-methylpiperazin-1-yl)methyl)-3-trifluoromethylphenyl)urea

The product (0.836 g, 1.7 mmol) obtained from Step 2, K₂CO₃ (0.704 g, 5.1 mmol) and MeOH (20 mL) were added into a 50 mL round-bottomed flask, and stirred at room temperature for 4 hours. The volatiles were distilled off under reduced pressure, and the residue was extracted with ethyl acetate (50 mL x 3) and H₂O (50 mL). The organic phases were combined, dried over anhydrous Na₂SO₄ and concentrated, to give a yellow solid.

### Step 4: Preparation of N-[3-((quinazolin-7-yl)ethynyl)-4-methyl]phenyl-N'-[4-((4-methylpiperazin-1-yl)methyl)-3-trifluoromethylphenyl)-yl]urea

The product (108 mg, 0.25 mmol) obtained from Step 3, 7-bromoquinazoline (62 mg, 0.30 mmol), Pd(PPh₃)₂Cl₂ (1.4 mg, 0.02 mmol), tricyclohexylphosphine (10 mg, 0.04 mmol), Cs₂CO₃ (49 mg, 0.15 mmol) and DBU (6d) and DMF (5 mL) were added into a 50 ml sealed tube, and stirred at 80°C for 48 hours under protection of argon gas. After heating was stopped, the system was cooled to room temperature, filtered, and extracted with ethyl acetate (30 mL x 3) and H₂O (30 mL). The organic phases were combined, dried over anhydrous Na₂SO₄, concentrated and purified by column chromatography, to give the title compound as a white viscous matter.

¹HNMR (500 MHz, CDCl₃) δ: 9.28 (m, 2H, 2×Ar-*H*), 8.10 (s, 1 H, Ar-*H*), 8.03 (s, 1 H, N-*H*), 7.93 (s, 1 H, N-*H*), 7.79-7.77 (d, 1 H, Ar-*H*), 7.59-7.45 (m, 5H, 5×Ar-*H*), 7.19-7.17 (m, 1 H, Ar-*H*), 7.05-7.03 (d, 1 H, Ar-*H*), 3.48 (s, 2H, PhC*H₂*), 2.40-2.36 (m, 11 H, *CH₃,* 4×-CH₂), 2.25 (s, 3H, C*H₃*).

ESI-MS *m*/*z*:[M + H]⁺ =559.3 (calculated: 559.6).

### Example 32: Preparation of N-[3-((3-amino-1H-indazol-4-yl)ethynyl)-4-methyl]phenyl-N'-[4-((4-methylpiperazin-1-yl)methyl)-3-trifluoromethylphenyl]urea

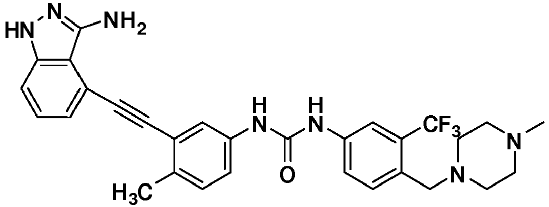

The title compound was prepared using N-(3-ethynyl-4-methyl)phenyl-N'-[4-(4-methylpiperazin-1-ylmethyl)-3-trifluoromethylphenyl]urea and 3-amino-4-bromo-1H-indazole according to the method described in Example 31.

¹H NMR (500 MHz, *d⁶*-DMSO) δ: 11.78 (s, 1 H, N-*H*), 9.04 (s, 1 H, Ar-*H*), 8.83 (s, 1 H, N-*H*), 7.96 (s, 1 H, N-*H*), 7.73-7.72 (d, 1 H, Ar-*H*), 7.63-7.61 (d, 1 H, Ar-*H*), 7.41-7.39 (m, 1 H, Ar-*H*), 7.34-7.33 (d, 1 H, Ar-*H*), 7.29-7.26 (t, 2H, Ar-*H*), 7.17-7.16 (d, 1 H, Ar-*H*), 5.13 (s, 2H, N*H*₂), 3.53 (s, 2H, NC*H*₂), 2.45 (s, 3H, C*H₃*), 2.38-2.20 (m, 8H, NC*H₂*C*H₂*N), 2.16 (s, 3H, *CH₃).*

MS: m/z [M + H]⁺ 562.3, calculated: 562.2.

### Example 33: In vitro evaluation of cell viability by the compounds

In this example, MTT assay was used to detect *in vitro* inhibitory activity of the compounds prepared according to the above examples on the cells. Imatinib and AP24534 were used as controls. Imatinib was prepared according to the method described in Chinese Patent No. CN1043531C and identified by 1H-NMR and MS. AP24534 was purchased from Shanghai Xinkuo Chemical Technology Co., Ltd., China.

The used cells included K562 leukemia cells, Saos-2 human osteosarcoma cells, Ovcar-3 human ovarian cancer cells and MDA-MB-231 human breast cancer cells, which all were purchased from Nanjing KeyGen Biotech. Co., Ltd.

Experimental principle: The detection principle is that succinate dehydrogenase in mitochondria of living cells is capable of reducing exogenous MTT into water-insoluble blue-violet crystal formazan and depositing in cells, whereas dead cells do not have the function. Dimethyl sulfoxide (DMSO) is capable of dissolving formazan in cells, and absorbance value can be measured at a wavelength of 490 nm by an enzyme-linked immunometric meter, which reflects the number of living cells. Within a certain range of the number of cells, the amount of MTT crystals formed is proportional to the number of living cells.

Experimental method:
1. Collecting the cells in logarithmic phase, adjusting the concentration of the cell suspension to about 1 x 10⁵ cells/ml, and inoculating into 96-well plates with 100 µl per well.
2. Culturing in a 37°C, 5% CO₂ incubator and keeping the cells adhering to the wall of the well.
3. Adding different concentrations of drug (the drug has been subjected to suitable treatment such as solubility, sterilization, etc.), and maintaining for an appropriate time period according to the experimental need, typically 48 hours.
4. Carefully removing the supernatant, gently washing with PBS and discarding the supernatant again.
5. Adding 180 µl fresh RPMI 1640 medium into each well, adding 20 µl MTT solution (5 mg/ml, that is, 0.5% MTT), and culturing for another 4 hours.
6. Terminating culturing and carefully discarding the medium in each well.
7. Adding 150 µl dimethyl sulfoxide into each well, shaking for 10 minutes at low speed in a shaker, to make crystals fully dissolved.
8. Measuring the absorbance of each well at 490 nm by an enzyme-linked immunometric meter.
9. Calculating IC₅₀ values. The experimental results were shown in Table 1.

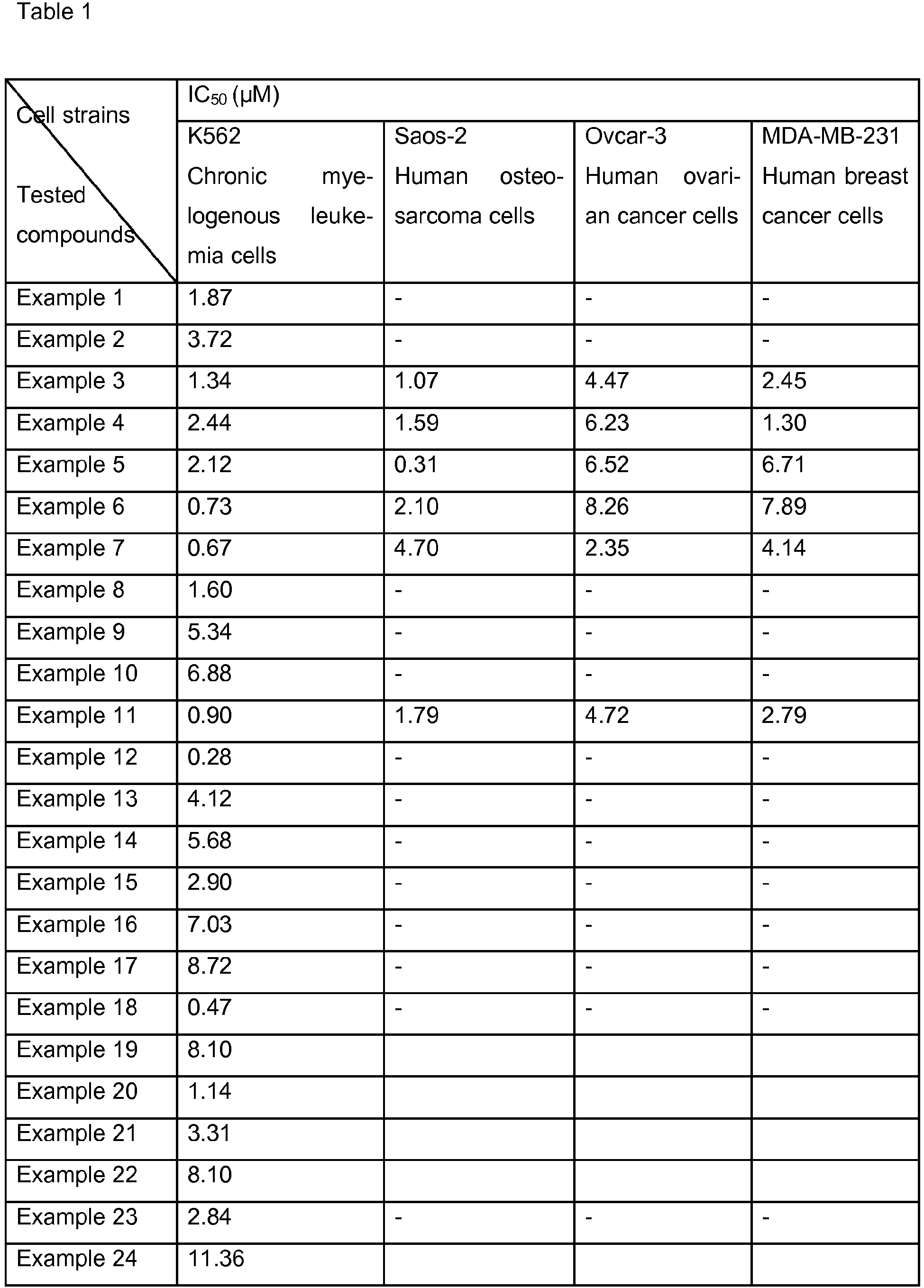

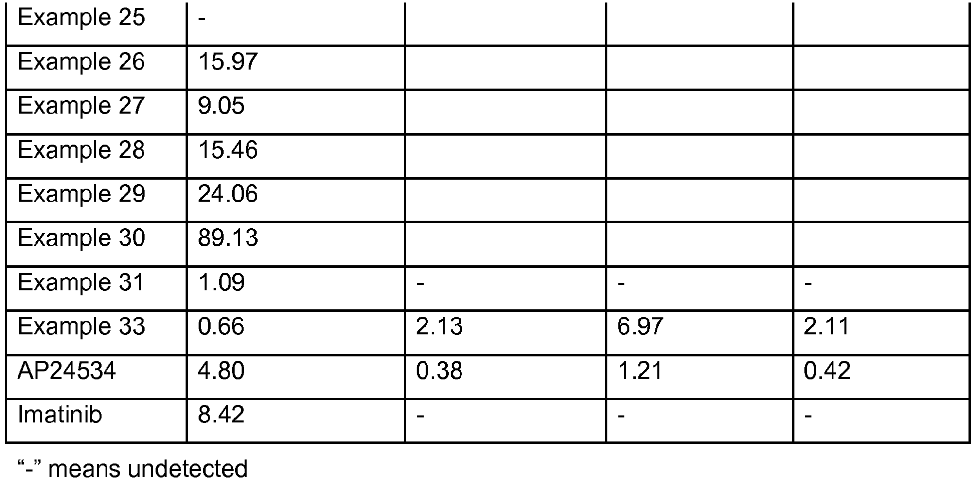

### Example 34: Evaluation of ABL1 (T315I) kinase activity by some compounds

In the experimental example, the compounds prepared according the examples of the present invention were tested for the ability to inhibit ABL (T315I) kinase activity. Imatinib was used as control.

A commercially available human ABL T315I mutant enzyme (Human ABL1 (T315I), active, catalog number # 14-522, Millipore Corporation, USA) was used to test ABL (T315I) tyrosine kinase activity. Kinase activity was determined according to the manufacturer's instructions. Peptide substrate is Abltide (EAIYAAPFAKKK), purchased from Millipore Corporation, USA. Ion exchange chromatography paper P81 was purchased from Whatman Company, UK. [γ-33P] ATP was purchased from Perkin Elmer Company.

Experimental protocol: Serially diluting the compounds of the present invention from 1 µM initial concentration in three-fold fashion and formulating 10 concentrations (50.8 pM, 152.0 pM, 457.0 pM, 1.37 nM, 4.12 nM, 12.3 nM, 37.0 nM, 111.0 nM, 333.0 nM and 1.0 µM). 5.0 µM Abltide was added into each well and then human T315I mutant enzyme was added. [γ-33P] ATP was added at room temperature, with final concentration of 1.0µM, and the reaction was performed for 120 minutes. 20 µl aliquots were transferred onto the ion exchange chromatography paper P81. The paper was thoroughly washed with a 0.75% phosphoric acid solution three times, and then washed with acetone once. Finally, γ-33P radioactivity was measured. The results were shown in Table 2 below.

**Table 2 BL1 (T315I) kinase activity data**

| Compounds | IC₅₀ (nM) |
|---|---|
| Example 1 | 6.48 |
| Example 3 | 4.51 |
| Example 4 | 2.30 |
| Example 9 | 5.80 |
| Example 12 | 31.68 |
| Example 13 | 9.50 |
| Example 16 | 12.36 |
| Example 18 | 21.73 |
| Example 20 | 43.50 |
| Example 21 | 24.30 |
| Example 23 | 6.00 |
| AP24534 | 1.00 |
| Imatinib | >1000 |

As shown by the above experimental results, the compounds of the present invention have IC₅₀ values for inhibiting T315I mutant enzyme significantly better than Imatinib, and are comparable in magnitude with AP24534. The compounds of the present invention have powerful inhibitory effect on T315I mutant enzymes.

### Example 35: In vitro evaluation of Bcr-Abl-positive cell activity by some compounds

### 1.1 Compounds

Each compound was dissolved in DMSO to 10 mM, diluted to 50 µM with complete medium, and then diluted to 10 µM with 0.1% DMSO in complete medium. The resulting solution was 10-fold serially diluted and formulated into 10 concentrations. Imatinib was used as positive control.

### 1.2 Cells

MEG-01 human megakaryocyte leukemia cells and KU812 human peripheral blood baso-philic leukocytes, purchased from ATCC Company, USA.

### 1.3 Reagents

Dimethyl sulfoxide (DMSO), purchased from Sigma Company, USA;
Luminescent cell viability assay kit (CellTiter-Glo^{®} Luminescent Cell Viability Assay Kit), purchased from Promega Corporation, USA;
Cell Titer-Glo® Substrate and Cell Titer-Glo® Buffer, purchased from Promega Corporation, USA;
IMEM medium, purchased from Gibco Company, USA;
RPMI 1640 medium, purchased from Gibco Company, USA;
Penicillin/streptomycin (Pen/Strep), purchased from Gibco Company, USA;
Fetal bovine serum (FBS), purchased from Gibco Company, USA;
0.25% trypsin-EDTA, purchased from Gibco Company, USA;
10 cm cell culture dish, purchased from Corning Corporation, USA;
50 mL centrifuge tube, purchased from Corning Corporation, USA;
384 well flat clear bottom white, purchased from Corning Corporation, USA;
Phosphate buffer saline (PBS), weekly prepared.

### 1.4 Instrument

PHERAstar Plus microplate reader, purchased from BMG Labtech Company, Germany.

### 2 Experimental methods:

### 3.3 Cell viability assay protocol

1) Collecting the cells in logarithmic phase, adjusting the concentration of the cell suspension to about 1 x 10⁵ cells/ml, and seeding into 384-well plates with 40 µl per well, that is, 4 x 10³ cells/well. The peripheral wells were filled with sterile PBS;
2) Adding 10 µl of 5 x concentration gradient of the compounds, and adding 10 µl medium containing 0.5% DMSO into the blank control wells, in which the concentration of DMSO was 0.1%;
3) Incubating the cells in a 37°C/5% CO₂ incubator;
4) Adding 30 µL Cell Titer-Glo^{®} Reagent at 72 hours after adding the compounds;
5) Incubating in a 37°C/5% CO₂ incubator for 10 minutes; and centrifuging at low speed and then measuring chemiluminescence values on a PHERAstar microplate reader.
6) Calculating cell viability (Cell Viability) = (RLUₛₐₘₚₗₑ/RLU_{negative}) x 100%, wherein RLUₛₐₘₚₗₑ was RLU (Relative Light Units) value of the well added with the compound and RLU_{negative} was RLU value of the well without the compound (that is, cell control, which was treated with the same concentration of DMSO). Data were processed by using a four-parameter logistic fitting module in Graphpad Prism 4.0 software to calculate IC₅₀. IC₅₀ value indicates the concentration of a compound inhibiting 50% of the cell growth, compared with the control group without adding the compounds. The experimental results were shown in Table 3 below.

**Table 3**

| Compounds | Cell strains IC₅₀ (nM) | |
|---|---|---|
| | MEG-01 | KU812 |
| Example 9 | 1.49 | 0.518 |
| Example 13 | 3.56 | 1.17 |
| Imatinib | 176 | 65.00 |

According to the above data, it can be seen that the compounds of the present invention have an activity on Bcr-Abl positive cell stains much better than Imatinib, and have stronger inhibitory effect.

From the above experimental results, it is concluded that the compounds of the present invention exhibit excellent effect on unmutated leukemia cells, especially have strong inhibition on Bcr-Abl positive cells, and meanwhile significantly inhibit the T315I mutant enzyme. Therefore, the compounds of the present invention are broad-spectrum Bcr-Abl inhibitors.

Although the present invention has been described in details above, it should be understood by persons of ordinary skill in the art that various modifications and alterations can be made without departing from the spirit and scope of the present invention. The scope of the invention is not limited to the foregoing detailed description and is defined by the appended claims.

## Claims

1. A compound of general formula I, 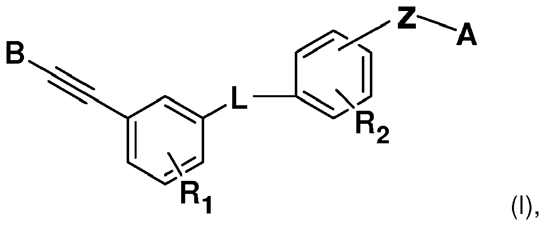 or a pharmaceutically acceptable salt, stereoisomer, N-oxide, solvate or prodrug thereof,
wherein
L is selected from -C(O)NH-, -NHC(O)NH- and -NHC(O)-;
Z is selected from (CH₂)ₙ and O, wherein n is selected from 0, 1, 2, 3 and 4;
A is selected from substituted and unsubstituted 5-, 6- and 7-membered nitrogen-containing heterocyclic groups;
R₁ is selected from H, alkyl, alkoxy, halo-substituted alkyl, halo-substituted alkoxy, - OH, -NH₂, halogen and -CN;
R₂ is selected from H, alkyl, alkoxy, halo-substituted alkyl, halo-substituted alkoxy, - OH, -NH₂, halogen and -CN; and
B is selected from groups represented by the formula 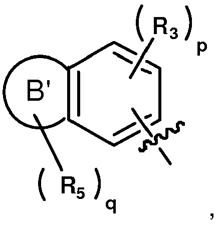 wherein B' is a 5- to 7-membered saturated or unsaturated heteroaromatic ring containing 1 to 3 nitrogen atoms;
R₃ is selected from H, alkyl, alkoxy, halo-substituted alkyl, halo-substituted alkoxy, - OH, -NH₂, halogen and -CN;
R₅ is selected from H, -NR₆R₇, -NHCOR₈, -SO₂R₈, alkyl, alkoxy, halo-substituted alkyl, halo-substituted alkoxy, -OH, halogen, oxo, -CN, hydrazino and alkyl substituted hydrazino, wherein R₆ and R₇ are independently selected from H, alkyl, 4-methylsulphonylanilino and 4-aminosulphonylanilino, and R₈ is selected from H and alkyl;
p is selected from 1, 2 and 3; and
q is selected from 1 and 2.

2. The compound according to claim 1, or the pharmaceutically acceptable salt, stereoisomer, N-oxide, solvate or prodrug thereof, wherein A is selected from piperazinyl, pyridinyl, imidazolyl, pyrrolidinyl, piperidinyl, morpholinyl, thiomorpholinyl, 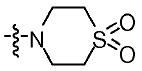 or substituted piperazinyl, pyridinyl, imidazolyl, pyrrolidinyl, piperidinyl, morpholinyl, thiomorpholinyl, wherein the substituent(s) is(are) selected from alkyl, hydroxy, hydroxyalkyl, alkoxy, amino, mono-alkylamino, di-alkylamino, amido, alkylamido, arylamido, heteroarylamido, halogen, halo-substituted alkyl, halo-substituted alkoxy and -CN, and B' is a 5-, 6- or 7-membered heteroaromatic ring containing 2 or 3 nitrogen atoms.

3. The compound according to claim 1, or the pharmaceutically acceptable salt, stereoisomer, N-oxide, solvate or prodrug thereof, wherein
L is selected from -C(O)NH-, -NHC(O)NH- and -NHC(O)-;
Z is selected from (CH₂)ₙ and O, wherein n is selected from 0, 1, 2, 3 and 4;
A is selected from substituted and unsubstituted 5-, 6- and 7-membered nitrogen-containing heterocyclic groups;
R₁ is selected from H, alkyl, alkoxy, halo-substituted alkyl, halo-substituted alkoxy, - OH, -NH₂, halogen and -CN;
R₂ is selected from H, alkyl, alkoxy, halo-substituted alkyl, halo-substituted alkoxy, - OH, -NH₂, halogen and -CN; and
B is selected from the following structures: 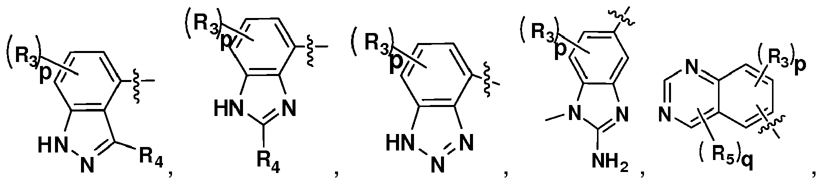 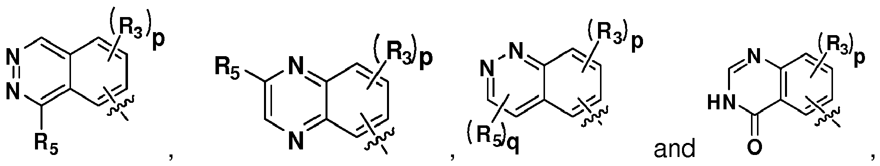 wherein R₃ is selected from H, alkyl, alkoxy, halo-substituted alkyl, halo-substituted alkoxy, -OH, -NH₂, halogen and -CN;
R₄ is -NH₂;
R₅ is selected from H, -NR₆R₇, -NHCOR₈, -SO₂R₈, alkyl, alkoxy, halo-substituted alkyl, halo-substituted alkoxy, -OH, halogen, -CN, hydrazino and alkyl substituted hydrazino, wherein R₆ and R₇ are independently selected from H, alkyl, 4-methylsulphonylanilino and 4-aminosulphonylanilino, and R₈ is selected from H and alkyl;
p is selected from 1, 2 and 3; and
q is selected from 1 and 2.

4. The compound according to any one of claims 1 to 3, or the pharmaceutically acceptable salt, stereoisomer, N-oxide, solvate or prodrug thereof, wherein when Z is O, A is pyridinyl or pyridinyl substituted by N-alkyl substituted carboxamido, preferably, A is pyridinyl, N-methylpicolinamido, N-ethylpicolinamido or N-propylpicolinamido, and further preferably, A is pyridin-4-yl, N-methylpicolinamido-4-yl, N-ethylpicolinamido-4-yl or N-propylpicolinamido-4-yl.

5. The compound according to any one of claims 1 to 3, or the pharmaceutically acceptable salt, stereoisomer, N-oxide, solvate or prodrug thereof, wherein when Z is selected from (CH₂)ₙ, A is selected from morpholinyl, thiomorpholinyl, 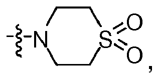 1 H-imidazolyl, 4-methyl-1 H-imidazolyl, piperidinyl, piperazinyl, pyrrolidinyl, and piperazinyl, pyrrolidinyl and piperidinyl substituted by one or more amino groups, C₁₋₆ alkyl groups, bi-C₁₋₆ alkylamino groups, mono-C₁₋₆ alkylamino groups or hydroxyethyl groups.

6. The compound according to claim 5, or the pharmaceutically acceptable salt, stereoisomer, N-oxide, solvate or prodrug thereof, wherein A is selected from morpholinyl, thiomorpholinyl, 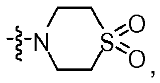 1 H-imidazolyl, 4-methyl-1 H-imidazolyl, piperazinyl, 4-methylpiperazinyl, 4-dimethylaminopiperazinyl, 4-methylaminopiperazinyl, 4-hydroxyethylpiperazinyl, 4-dimethylaminoethylpiperazinyl, piperidinyl, 4-dimethylaminopiperidinyl, pyrrolidinyl, 3-aminopyrrolidinyl, (R)-3-aminopyrrolidinyl, (S)-3-aminopyrrolidinyl, 3-dimethylaminopyrrolidinyl, (R)-3-dimethylaminopyrrolidinyl, (S)-3-dimethylaminopyrrolidinyl, 3-methylaminomethylpyrrolidinyl and 3-amino-3-methylpyrrolidinyl.

7. The compound according to any one of claims 1 to 3, or the pharmaceutically acceptable salt, stereoisomer, N-oxide, solvate or prodrug thereof, wherein R₁ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN; preferably R₁ is selected from H, C₁₋₃ alkyl, C₁₋₃ alkoxy, halo-substituted C₁₋₃ alkyl, halo-substituted C₁₋₃ alkoxy, -OH,-NH₂, halogen and -CN.

8. The compound according to any one of claims 1 to 3, or the pharmaceutically acceptable salt, stereoisomer, N-oxide, solvate or prodrug thereof, wherein R₂ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN; preferably R₂ is selected from H, C₁₋₃ alkyl, C₁₋₃ alkoxy, halo-substituted C₁₋₃ alkyl, halo-substituted C₁₋₃ alkoxy, -OH, -NH₂, halogen and -CN.

9. The compound according to any one of claims 1 to 3, or the pharmaceutically acceptable salt, stereoisomer, N-oxide, solvate or prodrug thereof, wherein R₃ is selected from H, C₁₋₆ alkyl, C₁₋₆ alkoxy, halo-substituted C₁₋₆ alkyl, halo-substituted C₁₋₆ alkoxy, -OH, -NH₂, halogen and -CN; preferably R₃ is selected from H, C₁₋₃ alkyl, C₁₋₃ alkoxy, halo-substituted C₁₋₃ alkyl, halo-substituted C₁₋₃ alkoxy, -OH, -NH₂, halogen and -CN.

10. The compound according to any one of claims 1 to 3, or the pharmaceutically acceptable salt, stereoisomer, N-oxide, solvate or prodrug thereof, wherein R₅ is selected from H, -NR₆R₇, -NHCOR₈, -SO₂R₈, C₁₋₆ alkyl, C₁₋₆ alkoxy, halo-substituted C₁₋₆ alkyl, halo-substituted C₁₋₆ alkoxy, -OH, -NH₂, halogen, -CN, hydrazino and C₁₋₆ alkyl substituted hydrazino; preferably R₅ is selected from H, -NR₆R₇, -NHCOR₈,-SO₂R₈, C₁₋₃ alkyl, C₁₋₃ alkoxy, halo-substituted C₁₋₃ alkyl, halo-substituted C₁₋₃ alkoxy, -OH, -NH₂, halogen, -CN, hydrazino and C₁₋₃ alkyl substituted hydrazino.

11. The compound according to any one of claims 1 to 3, or the pharmaceutically acceptable salt, stereoisomer, N-oxide, solvate or prodrug thereof, wherein R₆ and R₇ are independently selected from H, C₁₋₆ alkyl, 4-methylsulphonylanilino and 4-aminosulphonylanilino; and preferably, R₆ and R₇ are independently selected from H, C₁₋₃ alkyl, 4-methylsulphonylanilino and 4-aminosulphonylanilino.

12. The compound according to any one of claims 1 to 3, or the pharmaceutically acceptable salt, stereoisomer, N-oxide, solvate or prodrug thereof, wherein R₈ is selected from H and C₁₋₆ alkyl; preferably R₈ is selected from H and C₁₋₃ alkyl.

13. The compound according to any one of claims 1 to 3, or the pharmaceutically acceptable salt, stereoisomer, N-oxide, solvate or prodrug thereof, wherein the compound is selected from the following compounds:

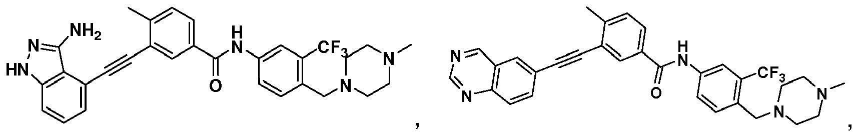

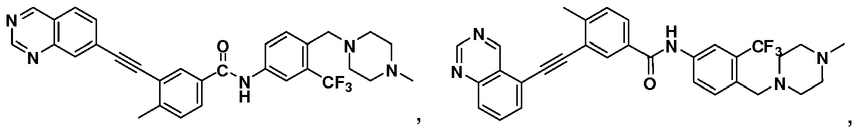

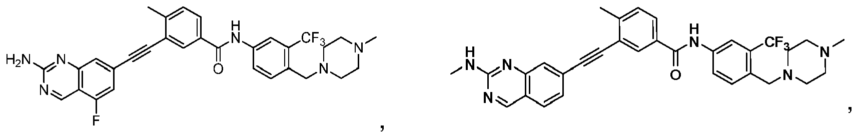

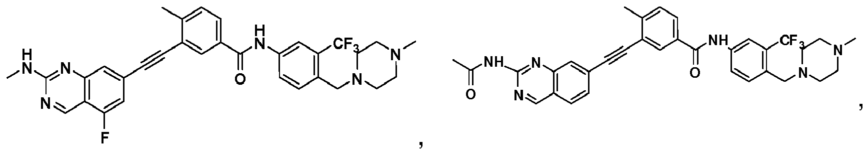

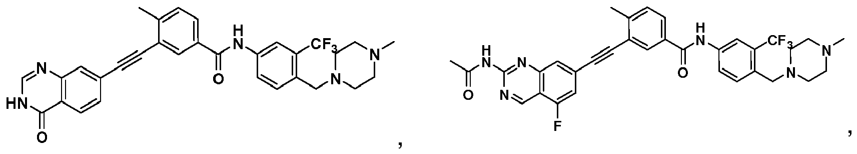

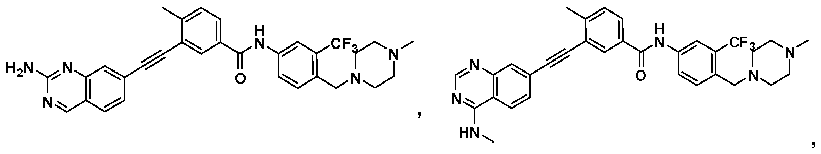

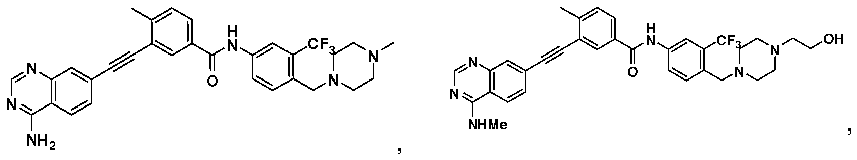

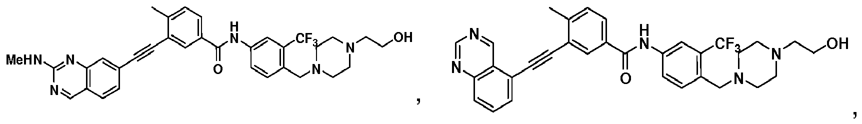

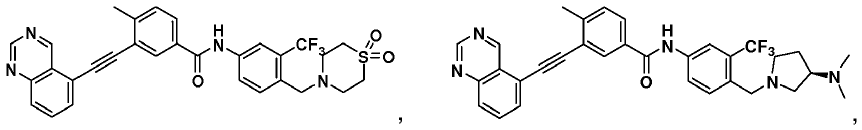

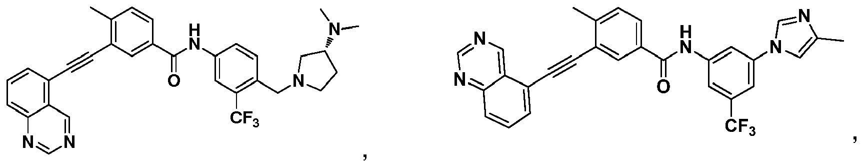

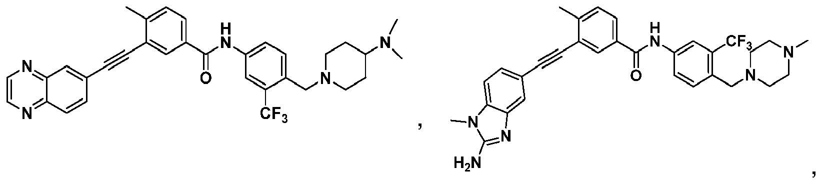

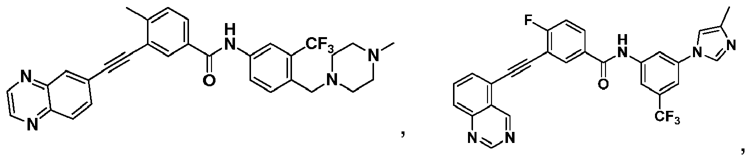

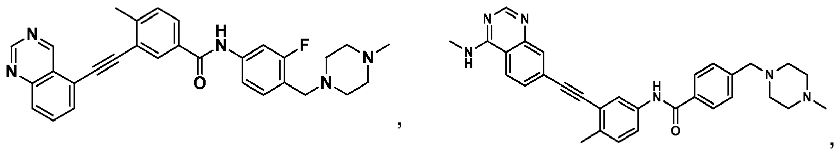

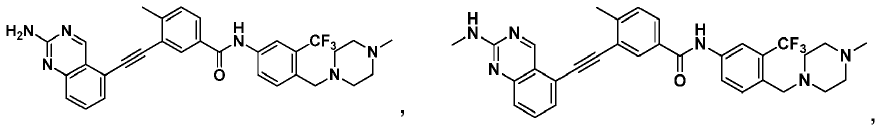

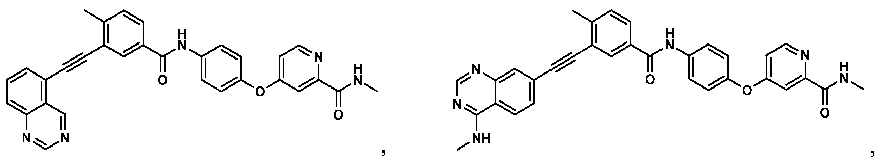

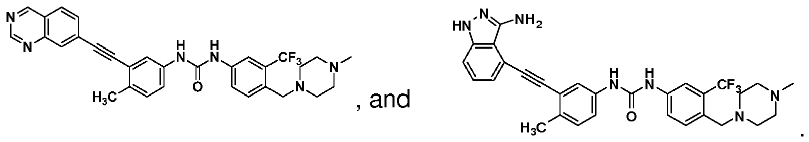

14. The compound according to any one of claims 1 to 13, or the pharmaceutically acceptable salt, stereoisomer, N-oxide, solvate or prodrug thereof, wherein the pharmaceutically acceptable salt is selected from the salts formed with the following acids: phosphoric acid, sulfuric acid, hydrochloric acid, hydrobromic acid, nitric acid, citric acid, maleic acid, malonic acid, mandelic acid, succinic acid, fumaric acid, acetic acid, lactic acid, sulfonic acid, oxalic acid, tartaric acid, *p*-toluenesulfonic acid, methanesulfonic acid, camphorsulfonic acid, gluconic acid, malic acid, palmitic acid, trifluoroacetic acid and amino acids.

15. A pharmaceutical composition comprising the compound according to any one of claims 1 to 14, or the pharmaceutically acceptable salt, stereoisomer, N-oxide, solvate or prodrug thereof, and a pharmaceutically acceptable carrier.

16. A use of the compound according to any one of claims 1 to 14, or the pharmaceutically acceptable salt, stereoisomer, N-oxide, solvate or prodrug thereof, or the pharmaceutical composition according to claim 15 in the manufacture of a medicament for treating and/or preventing tumors.
